# EUROPEAN PATENT SPECIFICATION

(11) **EP 1 884 581 B1**
(45) Date of publication and mention of the grant of the patent: **27.01.2010**
(21) Application number: 07019963.3
(22) Date of filing: 10.06.2004
(51) Int. Cl.: D01F 6/84, D01F 6/86, D01F 6/92, D01F 8/14

(54) **WATER-DISPERSIBLE AND MULTICOMPONENT FIBERS FROM SULFOPOLYESTERS**
WASSERDISPERGIERBARE MEHRKOMPONENTENFASER AUS SULFOPOLYESTERN
FIBRES DISPERSIBLES DANS L'EAU ET À MULTICOMPOSANTS PRODUITES DE SULFOPOLYESTERS

(30) Priority: 19.06.2003 US 465698; 20.05.2004 US 850548
(43) Date of publication of application: 06.02.2008
(62) Divisional of application: 04755058.7
(73) Proprietor: EASTMAN CHEMICAL COMPANY, Kingsport TN 37660 (US)
(72) Inventor: Haile, William Alston, Kingsport TN 37663 (US); Jenkins, Waylon Lewellyn, Kingsport TN 37664 (US); George, Scott Ellery, Kingsport TN 37664 (US); Hale, Wesley Raymond, Kingsport TN 37663 (US)
(74) Representative: Tostmann, Holger Carl

(56) References cited:
- WO-A1-93/07197
- US-A- 6 162 890
- PATENT ABSTRACTS OF JAPAN vol. 018, no. 236 (C-1196), 6 May 1994 (1994-05-06) & JP 06 025396 A (KURARAY CO LTD), 1 February 1994 (1994-02-01)
- PATENT ABSTRACTS OF JAPAN vol. 2000, no. 07, 29 September 2000 (2000-09-29) & JP 2000 095850 A (KANEBO LTD; KANEBO SYNTHETIC FIBERS LTD), 4 April 2000 (2000-04-04)

## Description

The present invention pertains to multicomponent fibers comprising a sulfopolyester yester and the microdenier fibers and fibrous articles prepared therefrom. The invention also pertains to processes for making, multicomponent, and microdenier fibers and to nonwoven fabrics prepared therefrom. The fibers and fibrous articles have applications In flushable personal care products and medical products.

### BACKGROUND OF THE INVENTION

Fibers, melt blown webs and other melt spun fibrous articles have been made from thermoplastic polymers, such as poly(propylene), polyamides, and polyesters. One common application of these fibers and fibrous articles are nonwoven fabrics and, in particular, in personal care products such as wipes, feminine hygiene products, baby diapers, adult incontinence briefs, hospital/surgical and other medical disposables, protective fabrics and layers, geotextiles, industrial wipes, and filter media. Unfortunately, the personal care products mades from conventional thermoplastic polymers are difficult to dispose of and are usually placed in landfills. One promising alternative method of disposal is to make these products or their components "flushable", i.e., compatible with public sewerage systems. The use of water-dispersible or water-soluble materials also improves recyclability and redamation of personal care products. The various thermoplastic polymers now used in personal care products are not inherently water-dispersible or soluble and, hence, do not produce articles that readily disintegrate and can be disposed of in a sewerage system or recycled easily.

The desirability of flushable personal care products has resulted in a need for fibers, nonwovens, and other fibrous articles with various degrees of water-responsivity. Various approaches to addressing these needs have been described, for example, in U.S. Patent No.'s 6,548,592; 6,552,162; 5,281,306; 5,292,581; 5,935,880; and 5,509,913; U.S. Patent Application Serial No.'s 09/775,312; and 09/752,017; and PCT International Publication No. WO 01/66666 A2. These approaches, however, suffer from a number of disadvantages and do not provide a fibrous article, such as a fiber or nonwoven fabric, that possesses a satisfactory balance of performance properties, such as tensile strength, absorptivity, flexibility, and fabric integrity under both wet or dry conditions.

For example, typical nonwoven technology is based on the multidirectional deposition of fibers that are treated with a resin binding adhesive to form a web having strong integrity and other desirable properties. The resulting assemblies, however, generally have poor water-responsivity and are not suitable for flushable applications. The presence of binder also may result in undesirable properties in the final product, such as reduced sheet wettability, increased stiffness, stickiness, and higher production costs. It is also difficult to produce a binder that will exhibit adequate wet strength during use and yet disperse quickly upon disposal. Thus, nonwoven assemblies using these binders may either disintegrate slowly under ambient conditions or have less than adequate wet strength properties in the presence of body fluids. To address this problem, pH and ion-sensitive water-dispersible binders, such as lattices containing acrylic or methacrylic acid with or without added salts, are known and described, for example, in U.S. Patent No. 6,548,592 B1. Ion concentrations and pH levels in public sewerage and residential septic systems, however, can vary widely among geographical locations and may not be sufficient for the binder to become soluble and disperse. In this case, the fibrous articles will not disintegrate after disposal and can dog drains or sewer laterals.

Multicomponent fibers containing a water-dispersible component and a thermoplastic water non-dispersible component have been described, for example, in U.S. Patent No.'s 5,916,678; 5,405,698; 4,966.808; 5,525282; 5,366,804; 5,486,418. For example, these multicomponent fibers may be a bicomponent fiber having a shaped or engineered transverse cross section such as, for example, an islands-in-the-sea, sheath core, side-by-side, or segmented pie configuration. The multicomponent fiber can be subjected to water or a dilute alkaline solution where the water-dispersible component is dissolved away to leave the water non-dispersible component behind as separate, independent fibers of extremely small fineness. Polymers which have good water dispersibility, however, often impart tackiness to the resulting multicomponent fibers, which causes the fiber to stick together, block, or fuse during winding or storage after several days, especially under hot, humid conditions. To prevent fusing, often a fatty add or oil-based finish is applied to the surface of the fiber. In addition, large proportions of pigments or fillers are sometimes added to water dispersible polymers to prevent fusing of the fibers as described, for example, in U.S. Patent No. 6,171,685. Such oil finishes, pigments, and fillers require additional processing steps and can impart undesirable properties to the final fiber. Many water-dispersible polymers also require alkaline solutions for their removal which can cause degradation of the other polymer components of the fiber such as, for example, reduction of inherent viscosity, tenacity, and melt strength. Further, some water-dispersible polymers can not withstand exposure to water during hydroentanglement and, thus, are not suitable for the manufacture of nonwoven webs and fabrics.

JP 10270748 (publication number 2000095850) relates to a copolyester containing ethylene terephthalate as main recurring unit and further (A) a metal sulfonate group-containing isophthalic acid (SIP) and (B) a polyalkylene glycol having 1,000 to 10,000 average molecular weight as copolymerization components. The object is to produce a polymer having good spinning properties, heat resistance and excellent spinning operation efficiency, while having sufficiently easy eluting properties in aqueous alkali solution.

JP 03353355 (publication number 06025396) relates to a polymer made up of dicarboxylic acid units, diol units and other units that can be obtained by polycondensation including, among others, a metal sulfonate containing comonomer. The purpose of providing that polymer is to obtain a copolyester that has a high surface wettability and results in excellent water absorption and water holding ability in the form of fibrous polymers.

US 6 162 890 relates to water-dispersible block copolyesters that are useful as low-odor adhesive materials. Disclosed in this context are linear, water-dispersible sulfopolysters encorporating a low molecular weight polyethylene glycol and blocks of high molecular weight polyethylene glycol. The blocks sulfopolyesters have improved toughness, abrasion resistance, flexibility and adhesion. Correspondingly, particular utility is seen in polyester fiber sizing applications and in adhesive applications.

WO 93/07197 relates to polyesters that are based upon a polyethylene terephthalate copolymerized with hexahydroterephthalic acid and containing di- or triethylene glycol and alkali metal or alkaline earth metal sulfo groups. These products are believed to be degradable under the conditions typically existing in waste composting processes and have low ingredient costs by providing strength and toughness properties adequate for end uses such as in disposable diapers.

Alternatively, the water-dispersible component may serve as a bonding agent for the thermoplastic fibers in nonwoven webs. Upon exposure to water, the fiber to fiber bonds come apart such that the nonwoven web loses its integrity and breaks down into individual fibers. The thermoplastic fiber components of these nonwoven webs, however, are not water-dispersible and remain present in the aqueous medium and, thus, must eventually be removed from municipal wastewater treatment plants. Hydroentanglement may be used to produce disintegratable nonwoven fabrics without or with very low levels (< 5 wt%) of added binder to hold the fibers together. Although these fabrics may disintegrate upon disposal, they often utilize fibers that are not water soluble or water-dispersible and may result in entanglement and plugging within sewer systems. Any added water-dispersible binders also must be minimally affected by hydroentangling and not form gelatinous buildup or cross-link, and thereby contribute to fabric handling or sewer related problems.

A few water-soluble or water-dispersible polymers are available, but are generally not applicable to melt blown fiber forming operations or melt spinning in general. Polymers, such as polyvinyl alcohol, polyvinyl pyrrolidone, and polyacrylic acid are not melt processable as a result of thermal decomposition that occurs at temperatures below the point where a suitable melt viscosity is attained. High molecular weight polyethylene oxide may have suitable thermal stability, but would provide a high viscosity solution at the polymer interface resulting in a slow rate of disintegration. Water-dispersible sulfopolyesters have been described, for example, in U.S. Patent No.'s 6,171,685; 5,543,488; 5,853,701; 4,304,901; 6,211,309; 5,570,605; 6,428,900; and 3.779,993. Typical sulfopolyesters, however, are low molecular weight thermoplastics that are brittle and lack the flexibility to withstand a winding operation to yield a roll of material that does not fracture or crumble. Sulfopolyesters also can exhibit blocking or fusing during processing into film or fibers, which may require the use of oil finishes or large amounts of pigments or fillers to avoid. Low molecular weight polyethylene oxide (more commonly known as polyethylene glycol) is a weak/brittle polymer that also does not have the required physical properties for fiber applications. Forming fibers from known water-soluble polymers via solution techniques is an alternative, but the added complexity of removing solvent, especially water, increases manufacturing costs.

Accordingly, adequate tensile strength, absorptivity, flexibility, and fabric integrity in the presence of moisture, especially upon exposure to human bodily fluids. In addition, a fibrous article is needed that does not require a binder and completely disperses or dissolves in residential or municipal sewerage systems. Potential uses include, but are not limited to, melt blown webs, spunbond fabrics, hydroentangled fabrics, dry-laid non-wovens, bicomponent fiber components, adhesive promoting layers, binders for cellulosics, flushable nonwovens and films, dissolvable binder fibers, protective layers, and there is a need for multicomponent fiber having a water-dispersible component that does not exhibit excessive blocking or fusing of filaments during spinning operations, is easily removed by hot water at neutral or slightly acidic pH, and is suitable for hydroentangling processes to manufacture nonwoven fabrics. Other extrudable and melt spun fibrous materials are also possible.

### SUMMARY OF THE INVENTION

We have unexpectedly discovered that flexible, water-dispersible fibers may be prepared from sulfopolyesters.
The water-dispersible, fibrous articles of the present invention include personal care articles such as, for example, wipes, gauze, tissue, diapers, training pants, sanitary napkins, bandages, wound care, and surgical dressings. In addition to being water-dispersible, the fibrous articles of our invention are flushable, that is, compatible with and suitable for disposal in residential and municipal sewerage systems.

The present invention provides a multicomponent fiber comprising a water-dispersible sulfopolyester and one or more water non-dispersible polymers. The fiber has an engineered geometry such that the water non-dispersible polymers are present as segments substantially isolated from each other by the intervening sulfopolyester, which acts as a binder or encapsulating matrix for the water non-dispersible segments. Thus, one aspect of our invention is a multicomponent fiber having a shaped cross section, comprising:
A) a water dispersible sulfopolyester having a glass transistion temperature (Tg) of at least 57°C, the sulfolyester comprising:
   (i) residues of one or more dicarboxylic acids;
   (ii)4 to 40 mole%, based on the total repeating units, of residues of at least one sulfomonomer having 2 functional groups and one or more sulfonate groups attached to an aromatic or cycloaliphatic ring wherein the functional groups are hydroxyl, carboxyl, or a combination thereof;
   (iii) one or more diol residues wherein at least 25 mole%, based on the total diol residues, is a poly(ethylene glycol) having a structure

      H-(OCH₂-CH₂)ₙ-OH

      wherein n is an integer in the range of 2 to 500; and
   (iv) 0 to 25 mole%, based on the total repeating units, of residues of a branching monomer having 3 or more functional groups wherein the functional groups are hydroxyl, carboxyl, or a combination thereof; and
B) a plurality of segments comprising one or more water-nondispersable polymers immiscible with the sulfopolyester,
wherein the segments are substantially isolated from each other by the sulfopolyester intervening between the segments;
wherein the fiber contains less than 10 weight percent of a pigment or filler, based on the total weight of the fiber.
The sulfopolyester has a glass transistion temperature of at least 57°C which greatly reduces blocking and fusion of the fiber during winding and long term storage. The sulfopolyester may be removed by contacting the multicomponent fiber with water to leave behind the water non-dispersible segments as microdenier fibers. Our invention, therefore, also provides a process for making microdenier fibers comprising:
A. spinning a water dispersible sulfopolyester having a glass transistion temperature (Tg) of at least 57°C and one or more water-nondispersable polymers immiscible with the sulfopolyester into multicomponent fibers, the sulfpolyester comprising:
   (i) 50 to 96 mole% of one or more residues of isophthalic acid or terephthalic acid, based on the total acid residues;
   (ii) 4 to 30 mole%, based on the total acid residues, of a residue of sodiosulfoisophthalic acid;
   (iii) one or more diol residues wherein at least 25 mole%, based on the total diol residues, is a poly(ethylene glycol) having a structure

      H-(OCH₂-CH₂)ₙ-OH

      wherein n is an integer in the range of 2 to 500; and
   (iv)0 to 20 mole%, based on the total repeating units, of residues of a branching monomer having 3 or more functional groups wherein the functional groups are hydroxyl, carboxyl, or a combination thereof;
   wherein the fibers have a plurality of segments comprising the water-nondispersable polymers wherein the segments are substantially isolated from each other by the sulfopolyester intervening between the segments and the fibers contain less than 10 weight percent of a pigment or filler, based on the total weight of the fibers; and
B. contacting the multicomponent fibers with water to remove the sulfopolyester thereby forming microdenier fibers.

The water non-dispersible polymers may be biodistintegratable as determined by DIN Standard 54900 and/or biodegradable as determined by ASTM Standard Method, D6340-98. The multicomponent fiber also may be used to prepare a fibrous article such as a yam, fabric, melt-blown web, spun-bonded web, or non-woven fabric and which may comprise one or more layers of fibers. The fibrous article having multicomponent fibers, in turn, may be contacted with water to produce fibrous articles containing microdenier fibers. Thus, another aspect of the invention is a process for making a microdenier fiber web, comprising:
A. spinning a water dispersible sulfopolyester having a glass transistion temperature (Tg) of at least 57°C and one or more water-nondispersable polymers immiscible with the sulfopolyester into multicomponent fibers, the sulfpolyester comprising:
   (i) 50 to 96 mole% of one or more residues of isophthalic acid or terephthalic acid, based on the total acid residues;
   (ii) 4 to 30 mole%, based on the total acid residues, of a residue of sodiosulfoisophthalic acid;
   (iii)one or more diol residues wherein at least 25 mole%, based on the total diol residues, is a poly(ethylene glycol) having a structure

      H-(OCH₂-CH₂)ₙ-OH

      wherein n is an integer in the range of 2 to 500; and
   (iv)0 to 20 mole%, based on the total repeating units, of residues of a branching monomer having 3 or more functional groups wherein the functional groups are hydroxyl, carboxyl, or a combination thereof.
   wherein the multicomponent fibers have a plurality of segments comprising the water-nondispersable polymers and the segments are substantially isolated from each other by the sulfopolyester intervening between the segments and the fibers contain less than 10 weight percent of a pigment or filler, based on the total weight of said fibers;
B. overlapping and collecting the multicomponent fibers of Step A to form a nonwoven web; and
C. contacting the nonwoven web with water to remove the sulfopolyester thereby forming a microdenier fiber web.

Our invention thus offers a novel and inexpensive process for making a water-dispersible nonwoven fabric by melt-spinning a water-dispersible sulfopolyester and forming a nonwoven web. The nonwoven fabric may be in the form of a flat fabric or a 3-dimensional shape and may be incorporated into a variety of fibrous articles such as the personal care articles noted hereinabove or used for the manufacture of water-dispersible and/or flushable protective outerware such as, for example, surgical gowns and protective clothing for chemical and biohazard cleanup and laboratory work.

### DETAILED DESCRIPTION

The present disclosure relates to water-dispersible fibers and fibrous articles that show tensile strength, absorptivity, flexibility, and fabric integrity in the presence of moisture, especially upon exposure to human bodily fluids. The fibers and fibrous articles of our invention do not require the presence of oil, wax, or fatty add finishes or the use of large amounts (typically 10 wt% or greater) of pigments or fillers to prevent blocking or fusing of the fibers during processing. In addition, the fibrous articles prepared from our novel fibers do not require a binder and readily disperse or dissolve in home or public sewerage systems. The fibers are water-dispersible fibers comprising a sulfopolyester having a glass transistion temperature (Tg) of at least 57° C, wherein the sulfpolyester comprises: (I) residues of one or more dicarboxylic acids; (ii) 4 to 40 mole%, based on the total repeating units, of residues of at least one sulfomonomer having 2 functional groups and one or more sulfonate groups attached to an aromatic or cycloaliphatic ring wherein the functional groups are hydroxyl, carboxyl, or a combination thereof; (iii) one or more diol residues wherein at least 25 mole%, based on the total diol residues, is a poly(ethylene glycol) having a structure

H-(OCH₂-CH₂)ₙ-OH

wherein n is an integer in the range of 2 to 500; and (iv) 0 to 25 mole%, based on the total repeating units, of residues of a branching monomer having 3 or more functional groups
wherein the functional groups are hydroxyl, carboxyl, or a combination thereof. Our fiber includes a water-non-dispersible polymer blended with the sulfopolyester with the proviso that the blend is an immiscible blend. Our fiber contains less than 10 weight percent of a pigment or filler, based on the total weight of the fiber. The present invention also includes fibrous articles comprising these fibers and may include personal care products such as wipes, gauze, tissue, diapers, adult incontinence briefs, training pants, sanitary napkins, bandages, and surgical dressings. The fibrous articles may have one or more absorbent layers of fibers.

Our invention provides multicomponent fibers, such as described, for example, in U.S. Patent No. 5,916,678, which may be prepared by extruding the sulfopolyester and one or more water non-dispersible polymers, which are immiscible with the sulfopolyester, separately through a spinneret having a shaped or engineered tranverse geometry such as, for example, an "islands-in-the-sea", sheath-core, side-by-side, or segmented pie configuration. The sulfopolyester may be later removed by dissolving the interfacial layers or pie segments and leaving the smaller filaments or microdenier fibers of the water non-dispersible polymer(s). For example, the sulfopolyester and water non dispersible polymers may be fed to a polymer distribution system where the polymers are introduced into a segmented spinneret plate. The polymers follow separate paths to the fiber spinneret and are combined at the spinneret hole which comprises either two concentric circular holes thus providing a sheath-core type fiber, or a circular spinneret hole divided along a diameter into multiple parts to provide a fiber having a side-by-side type. Alternatively, the immiscible water dispersible sulfopolyester and water non-dispersible polymers may be introduced separately into a spinneret having a plurality of radial channels to produce a multicomponent fiber having a segmented pie cross section. Typically, the sulfopolyester will form the "sheath" component of a sheath core configuration. In fiber cross sections having a plurality of segments, the water non-dispersible segments, typically, are substantially isolated from each other by the sulfopolyester. Alternatively, multicomponent fibers may be formed by melting the sulfopolyester and water non-dispersible polymers in separate extruders and directing the the polymer flows into one spinneret with a plurality of distribution flow paths in form of small thin tubes or segments to provide a fiber having an islands-in-the-sea shaped cross section. An example of such a spinneret is described in U.S. Patent No. 5,366,804. In the present invention, typically, the sulfopolyester will form the "sea" component and the water non-dispersible polymer will for the "islands" component.

Notwithstanding that the numerical ranges and parameters setting forth the broad scope of the invention are approximations, the numerical values set forth in the specific examples are reported as precisely as possible. Any numerical value, however, inherently contain certain errors necessarily resulting from the standard deviation found in their respective testing measurements.

The term "water-dispersibte", as used herein in reference to the sulfopolyester as one component of a multicomponent fiber or fibrous article, is intended to be synonymous with the terms "water-dissipatable", "water-disintegratable", "Water-dissolvable", "water-dispellable", "water soluble", "Water-removable", "hydro-soluble", and "hydrodispersible" and is intended to mean that the sulfopolyester component is sufficiently removed from the multicomponent fiber and is dispersed or dissolved by the action of water to enable the release and separation of the water non-dispersible fibers contained therein. The terms "dispersed", "dispersibe","dissipate", or "dissipatable" mean that, using a sufficient amount of deionized water (e.g., 100:1 water fiber by weight) to form a loose suspension or slurry of the fibers or fibrous article, at a temperature of 60°C, and within a time period of up to 5 days, sulfopolyester component dissolves, disintegrates, or separates from the multicomponent fiber, leaving behind a plurality of microdenier fibers from the water non-dispersible segments.

The sulfopolyesters of the present invention comprise dicarboxylic acid monomer residues, sulfomonomer residues, diol monomer residues, and repeating units. The sulfomonomer may be a dicarboxylic acid, a diol, or hydroxycarboxylic acid. Thus, the term "monomer residue", as used herein, means a residue of a dicarboxylic acid, a dial, or a hydroxycarboxylic acid. A "repeating unit", as used herein, means an organic structure having 2 monomer residues bonded through a carbonyloxy group. The sulfopolyesters of the present invention contain substantially equal molar proportions of acid residues (100 mole %) and diol residues (100 mole %) which react in substantially equal proportions such that the total moles of repeating units is equal to 100 mole %. The mole percentages provided in the present disclosure, therefore, may be based on the total moles of acid residues, the total moles of diol residues, or the total moles of repeating units. For example, a sulfopolyeseter containing 30 mole% of a sulfomonomer, which may be a dicarboxylic acid, a diol, or hydroxycarboxylic acid, based on the total repeating units, means that the sulfopolyester contains 30 mole% sulfomonomer out of a total of 100 mole% repeating units. Thus, there are 30 moles of sulfomonomer residues among every 100 moles of repeating units. Similarly, a sulfopolyeseter containing 30 mole% of a dicarboxylic acid sulfomonomer, based on the total acid residues, means the sulfopolyester contains 30 mole% sulfomonomer out of a total of 100 mole% acid residues. Thus, in this latter case there are 30 moles of sulfomonomer residues among every 100 moles of acid residues.

The sulfopolyesters described herein have an inherent viscosity, abbreviated hereinafter as "lh.V." of at least 0.1 dL/g, preferably 0.2 to 0.3 dUg, and most preferably greater than 0.3 dUg, measured in a 60/40 parts by weight solution of phenol/tetrachloroethane solvent at 25°C and at a concentration of 0.5 g of sulfopolyester in 100 mL of solvent. The term "polyester", as used herein, encompasses both "homopolyesters" and "copolyesters" and means a synthetic polymer prepared by the polycondensation of difunctional carboxylic acids with difunctional hydroxyl compound. As used herein, the term "sulfopolyester" means any polyester comprising a sulfomonomer. Typically the difunctional carboxylic acid is a dicarboxylic acid and the difunctional hydroxyl compound is a dihydric alcohol such as, for example glycols and diols. Alternatively, the difunctional carboxylic acid may be a hydroxy carboxylic acid such as, for example, p-hydroxybenzoic acid, and the difunctional hydroxyl compound may be a aromatic nucleus bearing 2 hydroxy substituents such as, for example, hydroquinone. The term "residue", as used herein, means any organic structure incorporated into the polymer through a polycondensation reaction involving the corresponding monomer. Thus, the dicarboxylic acid residue may be derived from a dicarboxylic acid monomer or its associated add halides, esters, salts, anhydrides, or mixtures thereof. As used herein, therefore, the term dicarboxylic acid is intended to include dicarboxylic acids and any derivative of a dicarboxylic acid, including its associated acid halides, esters, half-esters, salts, half-salts, anhydrides, mixed anhydrides, or mixtures thereof, useful in a polycondensation process with a diol to make a high molecular weight polyester.

The sulfopolyester of the present invention includes one or more dicarboxylic acid residues. Depending on the type and concentration of the sulfomonomer, the dicarboxylic acid residue may comprise from 60 to 100 mole% of the acid residues. Other examples of concentration ranges of dicarboxylic acid residues are from 60 mole% to 95 mole%, and 70 mole% to 95 mole%. Examples of dicarboxylic acids that may be used include aliphatic dicarboxylic acids, alicyclic dicarboxylic adds, aromatic dicarboxylic adds, or mixtures of two or more of these acids. Thus, suitable dicarboxylic adds include, but are not limited to succinic; glutaric; adipic; azelaic; sebacic; fumaric: maleic; itaconic; 1,3-cyclohexanedicarboxylic; 1,4-cyclohexanedicarboxylic; diglycolic; 2,5-nomomanedicarboxylic; phthalic; terephthalic; 1,4-naphthalenedicarboxylic; 2,5-naphthalenedicarboxylic; diphenic; 4,4-oxydibenzoic: 4,4'-sulfonyidibenzoic; and isophthalic. The preferred dicarboxylic acid residues are isophthalic, terephthalic, and 1,4-cyclohexanedicarboxylic acids, orifdiesters are used, dimethyl terephthalate, dimethyl isophthalate, and dimethyl-1,4-cyclohexanedicarboxylate with the residues of isophthalic and terephthalic acid being especially preferred. Although the dicarboxylic acid methyl ester is the most preferred embodiment, it is also acceptable to include higher order alkyl esters, such as ethyl, propyl, isopropyl, butyl, and so forth. In addition, aromatic esters, particularly phenyl, also may be employed.

The sulfopolyester includes 4 to 40 mole%, based on the total repeating units, of residues of at least one sulfomonomer having 2 functional groups and one or more sulfonate groups attached to an aromatic or cycloaliphatic ring wherein the functional groups are hydroxyl, carboxyl, or a combination thereof. Additional examples of concentration ranges for the sulfomonomer residues are 4 to 35 mole%, 8 to 30 mole%, and 8 to 25 mole%, based on the total repeating units. The sulfomonomer may be a dicarboxylic acid or ester thereof containing a sulfonate group, a diol containing a sulfonate group, or a hydroxy acid containing a sulfonate group. The term "sulfonate" refers to a salt of a sulfonic acid having the structure "-SO₃M" wherein M is the cation of the sulfonate salt. The cation of the sulfonate salt may be a metal ion such as Li⁺, Na⁺, K⁺, Mg⁺⁺, Ca⁺⁺, Ni⁺⁺, Fe⁺⁺, and the like. Alternatively, the cation of the sulfonate salt may be non-metallic such as a nitrogenous base as described, for example, in U.S. Patent No. 4,304,901. Nitrogen-based cations are derived from nitrogen-containing bases, which may be aliphatic, cycloaliphatic, or aromatic compounds. Examples of such nitrogen containing bases include ammonia, dimethylethanolamine, diethanolamane, triethanolamine, pyridine, morpholine, and piperidine. Because monomers containing the nitrogen-based sulfonate salts typically are not thermally stable at conditions required to make the polymers in the melt, the method of this invention for preparing sulfopolyesters containing nitrogen-based sulfonate salt groups is to disperse, dissipate, or dissolve the polymer containing the required amount of sulfonate group in the form of its alkali metal salt in water and then exchange the alkali metal cation for a nitrogen-based cation.

When a monovalent alkali metal ion is used as the cation of the sulfonate salt, the resulting sulfopolyester is completely dispersible in water with the rate of dispersion dependent on the content of sulfomonomer in the polymer, temperature of the water, surface area/thickness of the sulfopolyester, and so forth. When a divalent metal ion is used, the resulting sulfopolyesters are not readily dispersed by cold water but are more easily dispersed by hot water. Utilization of more than one counterion within a single polymer composition is possible and may offer a means to tailor or fine-tune the water-responsivity of the resulting article of manufacture. Examples sulfomonomers residues include monomer residues where the sulfonate salt group is attached to an aromatic acid nucleus, such as, for example, benzene; naphthalene; diphenyl; oxydiphenyl; sulfonyldiphenyl; and methylenediphenyl or cycloaliphatic rings, such as, for example, cyclohexyl; cyclopentyl; cyclobutyl; cycloheptyl; and cyclooctyl. Other examples of sulfomonomer residues which may be used in the present invention are the metal sulfonate salt of sulfophthalic acid, sulfoterephthalic acid, sulfoisophthalic acid, or combinations thereof Other examples of sulfomonomers which may be used are 5-sodiosulfoisophthalic acid and esters thereof. If the sulfomonomer residue is from 5-sodiosulfoisophthalic acid, typical sulfomonomer concentration ranges are 4 to 35 mole%, 8 to 30 mole %, and 8 to 25 mole %, based on the total moles of acid residues.

The sulfomonomers used in the preparation of the sulfopolyesters are known compounds and may be prepared using methods well known in the art. For example, sulfomonomers in which the sulfonate group is attached to an aromatic ring may be prepared by sulfonating the aromatic compound with oleum to obtain the corresponding sulfonic acid and followed by reaction with a metal oxide or base, for example, sodium acetate, to prepare the sulfonate salt Procedures for preparation of various sulfomonomers are described, for example, in U.S. Patent No.'s 3,779,993; 3,018,272; and 3,528.947.

It is also possible to prepare the polyester using, for example, a sodium sulfonate salt, and ion-exchange methods to replace the sodium with a different ion, such as zinc, when the polymer is in the dispersed form. This type of ion exchange procedure is generally superior to preparing the polymer with divalent salts insofar as the sodium salts are usually more soluble in the polymer reactant melt-phase.

The sulfopolyester includes one or more diol residues which may include aliphatic, cycloaliphatic, and aralkyl glycols. The cycloaliphatic diols, for example, 1,3-and 1,4-cyclohexanedimethanol, may be present as their pure cis or *trans* isomers or as a mixture of cis and *trans* isomers. As used herein, the term "diol" is synonymous with the term "glycol"and means any dihydric alcohol. Examples diols include ethylene glycol; diethylene glycol; triethylene glycol; polyethylene glycols; 1,3-propanediol; 2,4-dimethyl-2-ethylhexane-1.3-diol; 2,2-diinethyl-1,3-propanediol; 2-ethyl-2-butyl-1,3-propanediol; 2-ethyl-2-isobutyl-1,3-propanediol; 1,3-butanediol; 1,4-butanediol: 1,5-pentanediol; 1,6-hexanediol; 2.2.4-trimeth-1,6-hexanediol; thiodiethanol; 1,2-cydohexanedimethanol; 1,3-cydohexanedimethanol; 1,4-cyclohexanedimethanol; 2,2,4,4-tetramethyl-1,3-cyclobutanediol; p-xylylenediol, or combinations of one or more of these glycols.

The diol residues may include from 25 mole% to 100 mole%, based on the total diol residues, of residue of a poly(ethylene glycol) having a structure

H-(OCH₂-CH₂)ₙ-OH

wherein n is an integer in the range of 2 to 500. Non-limiting examples of lower molecular weight polyethylene glycols, e.g., wherein n is from 2 to 6, are diethylene glycol, triethylene glycol, and tetraethylene glycol. Of these lower molecular weight glycols, diethylene and triethylene glycol are most preferred. Higher molecular weight polyethylene glycols (abbreviated herein as "PEG"), wherein n is from 7 to 500, include the commercially available products known under the designation CARBOWAX®, a product of Dow Chemical Company (formerly Union Carbide). Typically, PEG's are used in combination with other diols such as, for example, diethylene glycol or ethylene glycol. Based on the values of n, which range from greater than 6 to 500. the molecular weight may range from greater than 300 to 22,000 g/mol. The molecular weight and the mole% are inversely proportional to each other, specifically, as the molecular weight is increased, the mole % will be decreased In order to achieve a designated degree of hydrophilicity. For example, it is illustrative of this concept to consider that a PEG having a molecular weight of 1000 may constitute up to 10 mole% of the total diol, while a PEG having a molecular weight of 10,000 would typically be incorporated at a level of less than 1 mole% of the total diol.

Certain dimer, trimer, and tetramer diols may be formed in situ due to side reactions that may be controlled by varying the process conditions. For example, varying amounts of diethylene, triethylene, and tetraethylene glycols may be formed from ethylene glycol from an acid-catalyzed dehydration reaction which occurs readily when the polycondensation reaction is carried out under acidic conditions. The presence of buffer solutions, well-known to those skilled in the art, may be added to the reaction mixture to retard these side reactions. Additional compositional latitude is possible, however, if the buffer is omitted and the dimerization, trimerization, and tetramerization reactions are allowed to proceed.

The sulfopolyester of the present invention may include from 0 to 25 mole%, based on the total repeating units, of residues of a branching monomer having 3 or more functional groups wherein the functional groups are hydroxyl, carboxyl, or a combination thereof. Non-limiting examples of branching monomers are 1,1,1-trimethylol propane, 1,1,1-trimethylolethane, glycerin, pentaerythritol, erythritol, threitol, dipentaerythritol, sorbitol, trimellitic anhydride, pyromellitic dianhydride, dimethylol propionic acid, or combinations thereof. Further examples of branching monomer concentration ranges are from 0 to 20 mole% and from 0 to 10 mole%. The presence of a branching monomer may result in a number of possible benefits to the sulfopolyester of the present invention, including but not limited to, the ability to tailor rheological, solubility, and tensile properties. For example, at a constant molecular weight, a branched sulfopolyester, compared to a linear analog, will also have a greater concentration of end groups that may facilitate post-polymerization crosslinking reactions. At high concentrations of branching agent, however, the sulfopolyester may be prone to gelation.

The sulfopolyester used for the fiber of the present invention has a glass transition temperature, abbreviated herein as "Tg", of at least 57 °C as measured on the dry polymer using standard techniques, such as differentical scanning calorimetry ("DSC"), well known to persons skilled in the art. The Tg measurements of the sulfopolyesters of the present invention are conducted using a "dry polymer", that is, a polymer sample In which adventitious or absorbed water is driven off by heating to polymer to a temperature of 200°C and allowing the sample to return to room temperature. Typically, the sulfopolyester is dried in the DSC apparatus by conducting a first thermal scan in which the sample is heated to a temperature above the water vaporization temperature, holding the sample at that temperature until the vaporization of the water absorbed in the polymer is complete (as indicated by an a large, broad endotherm), cooling the sample to room temperature, and then conducting a second thermal scan to obtain the Tg measurement.

The sulfopolyesters of this invention are blended with one or more supplemental polymers to modify the properties of the resulting fiber. The supplemental polymer is water-dispersible and is immiscible with the sulfopolyester. The term "miscible", as used herein, is intended to mean that the blend has a single, homogeneous amorphous phase as indicated by a single composition-dependent Tg. For example, a first polymer that is miscible with second polymer may be used to "plasticize" the second polymer as illustrated, for example, in U.S. Patent No. 6,211,309. By contrast, the term "immiscible", as used herein, denotes a blend that shows at least 2, randomly mixed, phases and exhibits more than one Tg. Some polymers may be immiscible and yet compatible with the sulfopolyester. A further general description of miscible and immiscible polymer blends and the various analytical techniques for their characterization may be found in Polymer Blends Volumes 1 and 2, Edited by D.R. Paul and C.B. Bucknall, 2000, John Wiley & Sons, Inc.

Examples of polymers which are water-nondispersible that may be blended with the sulfopolyester include, but are not limited to, polyolefins, such as homo- and copolymers of polyethylene and polypropylene; poly(ethylene terephthalate); poly(butylene terephthalate); and polyamides, such as nylon-6; polylactides; caprolactone; Eastar Bio^{®} (poly(tetramethylene adipate-co-terephthalate), a product of Eastman Chemical Company); polycarbonate; polyurethane; and polyvinyl chloride.

The blends of one or more sulfopolyesters will have Tg's of at least 57°C for the multicomponent fibers of the present invention. Thus, blending may also be exploited to alter the processing characteristics of a sulfopolyester to facilitate the fabrication of a nonwoven. In an example of the present invention, an immiscible blend of polypropylene and sulfopolyester may provide a conventional nonwoven web that will break apart and completely disperse in water as true solubility is not needed. In this latter example, the desired performance is related to maintaining the physical properties of the polypropylene while the sulfopolyester is only a spectator during the actual use of the product or, alternatively, the sulfopolyester is fugitive and is removed before the final form of the product is utilized.

The sulfopolyester and supplemental polymer may be blended in batch, semicontinuous, or continuous processes. Small scale batches may be readily prepared in any high-intensity mixing devices well-known to those skilled in the art, such as Banbury mixers, prior to melt-spinning fibers. The components may also be blended in solution in an appropriate solvent. The melt blending method includes blending the sulfopolyester and supplemental polymer at a temperature sufficient to melt the polymers. The blend may be cooled and pelletized for further use or the melt blend can be melt spun directly from this molten blend into fiber form. The term "melt" as used herein includes, but is not limited to, merely softening the polyester. For melt mixing methods generally known in the polymers art, see Mixing and Compounding of Polymers (I. Manas-Zloczower & Z. Tadmor editors, Carl Hanser Verlag Publisher, 1994, New York, N. Y.).

The sulfopolyesters of the instant invention are readily prepared from the appropriate dicarboxylic acids, esters, anhydrides, or salts, sulfomonomer, and the appropriate diol or diol mixtures using typical polycondensation reaction conditions. They may be made by continuous, semi-continuous, and batch modes of operation and may utilize a variety of reactor types. Examples of suitable reactor types include, but are not limited to, stirred tank, continuous stirred tank, slurry, tubular, wiped-film, failing film, or extrusion reactors. The term "continuous" as used herein means a process wherein reactants are introduced and products withdrawn simultaneously in an uninterrupted manner. By "continuous" it is meant that the process is substantially or completely continuous in operation and is to be contrasted with a "batch" process. "Continuous" is not meant in any way to prohibit normal interruptions in the continuity of the process due to, for example, start-up, reactor maintenance, or scheduled shut down periods. The term "batch" process as used herein means a process wherein all the reactants are added to the reactor and then processed according to a predetermined course of reaction during which no material is fed or removed into the reactor. The term "semicontinuous" means a process where some of the reactants are charged at the beginning of the process and the remaining reactants are fed continuously as the reaction progresses. Alternatively, a semicontinuous process may also include a process similar to a batch process in which all the reactants are added at the beginning of the process except that one or more of the products are removed continuously as the reaction progresses. The process is operated advantageously as a continuous process for economic reasons and to produce superior coloration of the polymer as the sulfopolyester may deteriorate in appearance if allowed to reside in a reactor at an elevated temperature for too long a duration.

The sulfopolyesters of the present invention are prepared by procedures known to persons skilled in the art. The sulfomonomer is most often added directly to the reaction mixture from which the polymer is made, although other processes are known and may also be employed, for example, as described in U. S. Patent No.'s 3,018,272, 3,075,952, and 3,033,822. The reaction of the sulfomonomer, diol component and the dicarboxylic acid component may be carried out using conventional polyester polymerization conditions. For example, when preparing the sulfopolyesters by means of an ester interchange reaction, i.e., from the ester form of the dicarboxylic add components, the reaction process may comprise two steps. In the first step, the diol component and the dicarboxylic acid component, such as, for example, dimethyl Isophthalate, are reacted at elevated temperatures, typically, 150°C to 250°C for 0.5 to 8 hours at pressures ranging from 0.0 kPa gauge to 414 kPa gauge (60 pounds per square inch, "psig"). Preferably, the temperature for the ester interchange reaction ranges from 180°C to 230°C for 1 to 4 hours while the preferred pressure ranges from 103 kPa gauge (15 psig) to 276 kPa gauge (40 psig). Thereafter, the reaction product is heated under higher temperatures and under reduced pressure to form sulfopolyester with the elimination of diol, which is readily volatilized under these conditions and removed from the system. This second step, or polycondensation step, is continued under higher vacuum and a temperature which generally ranges from 230°C. to 350°C, preferably 250°C to 310°C and most preferably 260°C to 290°C for 0.1 to 6 hours, or preferably, for 0.2 to 2 hours, until a polymer having the desired degree of polymerization, as determined by inherent viscosity, is obtained. The polycondensation step may be conducted under reduced pressure which ranges from 53 kPa (400 torr) to 0.013 kPa (0.1 torr). Stirring or appropriate conditions are used in both stages to ensure adequate heat transfer and surface renewal of the reaction mixture. The reactions of both stages are facilitated by appropriate catalysts such as, for example, alkoxy titanium compounds, alkali metal hydroxides and alcoholates, salts of organic carboxylic acids, alkyl tin compounds, metal oxides, and the like. A three-stage manufacturing procedure, similar to that described in U.S. Patent No. 5,290,631, may also be used, particularly when a mixed monomer feed of acids and esters is employed.

To ensure that the reaction of the diol component and dicarboxylic acid component by an ester interchange reaction mechanism is driven to completion, it is preferred to employ 1.05 to 2.5 moles of diol component to one mole dicarboxylic acid component. Persons of skill in the art will understand, however, that the ratio of diol component to dicarboxylic acid component is generally determined by the design of the reactor in which the reaction process occurs.

In the preparation of sulfopolyester by direct esterification, i.e., from the acid form of the dicarboxylic acid component, sulfopolyesters are produced by reacting the dicarboxylic acid or a mixture of dicarboxylic acids with the diol component or a mixture of diol components. The reaction is conducted at a pressure of from 7 kPa gauge (1 psig) to 1379 kPa gauge (200 psig), preferably less than 689 kPa (100 psig) to produce a low molecular weight, linear or branched sulfopolyester product having an average degree of polymerization of from 1.4 to 10. The temperatures employed during the direct esterification reaction typically range from 180°C to 280°C, more preferably ranging from 220°C to 270°C. This low molecular weight polymer may then be polymerized by a polycondensation reaction.

The multicomponent fibers and fibrous articles of this invention also may contain other conventional additives and ingredients which do not deleteriously affect their end use. For example, additives such as fillers, surface friction modifiers, light and heat stabilizers, extrusion aids, antistatic agents, colorants, dyes, pigments, fluorescent brighteners, antimicrobials, anticounterfeiting markers, hydrophobic and hydrophilic enhancers, viscosity modifiers, slip agents, tougheners, adhesion promoters, and the like may be used. The fibers and fibrous articles of our invention do not require the presence of additives such as, for example, pigments, fillers, oils, waxes, or fatty acid finishes, to prevent blocking or fusing of the fibers during processing. The terms "blocking or fusing", as used herein, is understood to mean that the fibers or fibrous articles stick together or fuse into a mass such that the fiber cannot be processed or used for its intended purpose. Blocking and fusing can occur during processing of the fiber or fibrous article or during storage over a period of days or weeks and is exacerbated under hot, humid conditions. In one embodiment of the invention, the fibers and fibrous articles will contain less than 10 wt% of such anti-blocking additives, based on the total weight of the fiber or fibrous article. For example, the fibers and fibrous articles may contain less than 10 wt% of a pigment or filler. In other examples, the fibers and fibrous articles may contain less than 9 wt%, less than 5 wt%, less than 3 wt%, less than 1 wt%, and 0 wt% of a pigment or fitter, based on the total weight of the fiber. Colorants, sometimes referred to as toners, maybe added to impart a desired neutral hue and/or brightness to the sulfopolyester. When colored fibers are desired, pigments or colorants may be included in the Sulfopolyester reaction mixture during the reaction of the diol monomer and the dicarboxylic acid monomer or they may be melt blended with the preformed sulfopolyester. A preferred method of including colorants is to use a colorant having thermally stable organic colored compounds having reactive groups such that the colorant is copolymerized and incorporated into the sulfopolyester to improve its hue. For example, colorants such as dyes possessing reactive hydroxyl and/or carboxyl groups, including, but not limited to, blue and red substituted anthraquinones, may be copolymerized into the polymer chain. When dyes are employed as colorants, they may be added to the copolyester reaction process after an ester interchange or direct esterification reaction.

For the purposes of this invention, the term "fiber" refers to a polymeric body of high aspect ratio capable of being formed into two or three dimensional articles such as woven or nonwoven fabrics. In the context of the present invention, the term "fiber" is synonymous with "fibers" and intented to mean one or more fibers. The fibers of our invention are multicomponent fibers. The term "multicomponent fiber", as used herein, intended to mean a fiber prepared by melting the two or more fiber forming polymers in separate extruders and by directing the resulting multiple polymer flows into one spinneret with a plurality of distribution flow paths but spun together to form one fiber. Multicomponent fibers are also sometimes referred to as conjugate or bicomponent fibers. The polymers are arranged in substantially constantly positioned distinct zones across the cross-section of the conjugate fibers and extend continuously along the length of the conjugate fibers. The configuration of such a multicomponent fiber may be, for example, a sheath/core arrangement wherein one polymer is surrounded byanotherormay be a side by side arrangement, a pie arrangement or an "islands-in-the-sea" arrangement. For example, a multicomponent fiber may be prepared by extruding the sulfopolyester and one or more water non-dispersible polymers separately through a spinneret having a shaped or engineered tranverse geometry such as, for example, an "islands-in-the-sea" or segmented pie configuration.

The multifilament fibers of our invention will preferably range in size from 1.5 micrometers for melt blown webs, 0.5 to 50 d/f (denier per-filament) for staple fibers, and up to 5000 d/f for monofilament fibers. Multifilament fibers may also be used as crimped or uncrimped yarns and tows. Fibers used in melt blown web and melt spun fabrics may be produced in microdenier sizes. The term "microdenier", as used herein, is intended to mean a d/f value of 1 d/f or less. For example, the microdenier fibers of the instant invention typically have d/f values of 1 or less, 0.5 or less, or 0.1 or less. Nanofibers can also be produced by electrostatic spinning.

As noted hereinabove, the sulfopolyesters are advantageous for the preparation of bicomponent and multicomponent fibers having a shaped cross section. We have discovered that sulfopolyesters or blends of sulfopolyesters having a glass transistion temperature (Tg) of at least 57°C are particularly useful for multicomponent fibers to prevent blocking and fusing of the fiber during spinning and take up. Thus, our invention provides a multicomponent fiber having shaped cross section, comprising:
A) a water dispersible sulfopolyester having a glass transistion temperature (Tg) of at least 57°C, the sulfpolyester comprising:
   (i) residues of one or more dicarboxylic acids;
   (ii) 4 to 40 mole%, based on the total repeating units, of residues of at least one sulfomonomer having 2 functional groups and one or more sulfonate groups attached to an aromatic or cycloaliphatic ring wherein the functional groups are hydroxyl, carboxyl, or a combination thereof;
   (iii)one or more diol residues wherein at least 25 mole%, based on the total diol residues, is a poly(ethylene glycol) having a structure

      H-(OCH₂-CH₂)ₙ-OH

      wherein n is an integer in the range of 2 to 500; and
   (iv) 0 to 25 mole%, based on the total repeating units, of residues of a branching monomer having 3 or more functional groups wherein the functional groups are hydroxyl, carboxyl, or a combination thereof; and
B) a plurality of segments comprising one or more water-nondispersable polymers immiscible with the sulfopolyester, wherein the segments are substantially isolated from each other by the sulfopolyester intervening between the segments;
wherein the fiber has an islands-in-the-sea or segmented pie cross section and contains less than 10 weight percent of a pigment or filter, based on the total weight of the fiber.
The dicarboxylic acids, diols, sulfopolyester, sulfomonomers, and branching monomers residues are as described previously. For multicomponent fibers, it is advantageous that the sulfopolyester have a Tg of at least 57°C. Further examples of glass transition temperatures that may be exhibited by the sulfopolyester or sulfopolyester blend of our multicomponent fiber are at least 60°C, at least 65°C, at least 70°C, at least 75°C, at least 80°C, at least 85°C, and at least 90°C. Further, to obtain a sulfopolyester with a Tg of at least 57°C, blends of one or more sulfopolyesters may be used in varying proportions to obtain a sulfopolyester blend having the desired Tg. The Tg of a sulfopolyester blend may be calculated by using a weighted average of the Tg's of the sulfopolyester components. For example, sulfopolyester having a Tg of 48°C may be blended in a 25:75 wt:wt ratio with another sulfopolyester having Tg of 65°C to give a sulfopolyester blend having a Tg of approximately 61°C.

The multicomponent fiber comprises a plurality of segments one or more water-nondispersable polymers immiscible with the sulfopolyester in which the segments are substantially Isolated from each other by the sulfopolyester intervening between the segments. The term "substantially isolated", as used herein, is intended to mean that the segments are set apart from each other to permit the segments to form individual fibers upon removal of the sulfopolyester. For example, the segments may be touching each others as in, for example, a segmented pie configuration but can be split apart by impact or when the sulfopolyester is removed.

The ratio by weight of the sulfopolyester to water non-dispersible polymer component in the multicomponent fiber of the invention is generally in the range of 60:40 to 2:98 or, in another example, in the range of 50:50 to 5:95. Typically, the sulfopolyester comprises 50% by weight or less of the total weight of the mulicomponenf fiber.

The segments of multicomponent fiber may comprise one of more water non-dispersible polymers. Examples of water-nondispersible polymers which may be used in segments of the multicomponent fiber include, but are not limited to, polyolefins, polyesters, polyamides, polylactides, polycaprolactone, polycarbonate, polyurethane, and polyvinyl chloride. For example, the water non-dispersible polymer may be polyester such as poly(ethylene) terephthalate, poly(butylene) terephthalate, poly(cyclohexylene) cyclohexanedicarboxylate, poly(cyclohexylene) terephthalate, poly(trimethylene) terephthalate, and the like. In another example, the water-nondispersible polymer biodistintegratable as determined by DIN Standard 54900 and/or biodegradable as determined by ASTM Standard Method, D6340-98. Examples of biodegradable polyesters and polyester blends are disclosed in U.S. Patent No.'s 5,599,858; 5,580,911; 5,446,079; and 5,559,171. The term "biodegradable", as used herein in reference to the water non-dispersible polymers of the present invention, is understood to mean that the polymers are degraded under environmental influences such as, for example, in a composting environment, in an appropriate and demonstrable time span as defined, for example, by ASTM Standard Method, D6340-98, entitled "Standard Test Methods for Determining Aerobic Biodegradation of Radiolabeled Plastic Materials in an Aqueous or Compost Environment". The water non-dispersible polymers of the present invention also may be "biodisintegratable", meaning that the polymers are easily fragmented in a composting environment as defined, for example, by DIN Standard 54900. For example, the biodegradable polymer is initially reduced in molecular weight in the environment by the action of heat, water, air, microbes and other factors. This reduction in molecular weight results in a loss of physical properties (tenacity) and often in fiber breakage. Once the molecular weight of the polymer is sufficiently low, the monomers and oligomers are then assimilated by the microbes, In an aerobic environment, these monomers or oligomers are ultimately oxidized to CO₂, H₂O and new cell biomass. In an anaerobic environment, the monomers or oligomers are ultimately converted to CO₂ H₂, acetate, methane, and cell biomass.

For example, water-nondispersible polymer may be an aliphatic-aromatic polyester, abbreviated herein as "AAPE". The term "aliphatic-aromatic polyester", as used herein, means a polyester comprising a mixture of residues from aliphatic or cycloaliphatic dicarboxylic acids or diols and aromatic dicarboxylic acids or diols. The term "non-aromatic", as used herein with respect to the dicarboxylic acid and diol monomers of the present invention, means that carboxyl or hydroxyl groups of the monomer are not connected through an aromatic nucleus. For example, adipic acid contains no aromatic nucleus in its backbone, i.e., the chain of carbon atoms connecting the carboxylic acid groups, thus is "non-aromatic". By contrast, the term "aromatic" means the dicarboxylic acid or diol contains an aromatic nucleus in the backbone such as, for example, terephthalic acid or 2,6-naphthalene dicarboxylic acid "Non-aromatic", therefore, is intended to include both aliphatic and cycloaliphatic structures such as, for example, diols and dicarboxylic acids, which contain as a backbone a straight or branched chain or cyclic arrangement of the constituent carbon atoms which may be saturated or paraffinic in nature, unsaturated, i.e., containing non-aromatic carbon-carbon double bonds, or acetylenic, i.e., containing carbon-carbon triple bonds. Thus, in the context of the description and the claims of the present invention, non-aromatic is intended to include linear and branched, chain structures (referred to herein as "aliphatic") and cyclic structures (referred to herein as "alicyclic" or "cycloaliphatic"). The term "non-aromatic", however, is not intended to exclude any aromatic substituents which may be attached to the backbone of an aliphatic or cycloaliphatic diol or dicarboxylic add. In the present invention, the difunctional carboxylic acid typically is a aliphatic dicarboxylic acid such as, for example, adipic acid, or an aromatic dicarboxylic acid such as, for example, terephthalic acid. The difunctional hydroxyl compound may be cycloaliphatic diol such as, for example, 1,4-cyclohexanedimethanol, a linear or branched aliphatic diol such as, for example, 1,4-butanediol, or an aromatic diol such as, for example, hydroquinone.

The AAPE may be a linear or branched random copolyester and/or chain extended copolyester comprising diol residues which comprise the residues of one or more substituted or unsubstituted, linear or branched, diols selected from aliphatic diols containing 2 to 8 carbon atoms, polyalkylene ether glycols containing 2 to 8 carbon atoms, and cycloaliphatic diols containing 4 to 12 carbon atoms. The substituted diols, typically, will comprise 1 to 4 substituents independently selected from halo, C₆-C₁₀ aryl, and C₁-C₄ alkoxy. Examples of diols which may be used include, but are not limited to, ethylene glycol, diethylene glycol, propylene glycol, 1,3-propanediol, 2,2-dimethyl-1,3-propanediol, 1,3-butanediol, 1,4-butanediol, 1,5-pentanediol, 1,6-hexanediol, polyethylene glycol, diethylene glycol, 2,2,4-trimethyl-1,6-hexanediol, thiodiethanol, 1,3-cyclohexanedimethanol, 1,4-cyclohexanedimethanol, 2,2,4,4-tetramethyl-1,3-cyclobutanediol, triethylene glycol, and tetraethylene glycol with the preferred diols comprising one or more diols selected from 1,4-butanediol; 1,3-propanediol; ethylene glycol; 1,6-hexanediol; diethylene glycol; or 1,4-cyclohexanedimethanol. The AAPE also comprises diacid residues which contain 35 to 99 mole%, based on the total moles of diacid residues, of the residues of one or more substituted or unsubstituted, linear or branched, non-aromatic dicarboxylic acids selected from aliphatic dicarboxylic acids containing 2 to 12 carbon atoms and cycloaliphatic adds containing 5 to 10 carbon atoms. The substituted non-aromatic dicarboxylic acids will typically contain 1 to 4 substituents selected from halo, C₆-C₁₀ aryl, and C₁-C₄ alkoxy. Non-limiting examples of non-aromatic diacids include malonic, succinic, glutaric, adipic, pimelic, azelaic, sebacic, fumaric, 2,2-dimethyl glutaric, suberic, 1,3-cyclopentanedicarboxylic, 1,4-cyclohexanedicarboxylic, 1,3-cyclohexanedicarboxylic, diglycolic, itaconic, maleic, and 2,5-norbomanedicarboxylic. In addition to the non-aromatic dicarboxylic acids, the AAPE comprises 1 to 65 mole%, based on the total moles of diacid residues, of the residues of one or more substituted or unsubstituted aromatic dicarboxylic acids containing 6 to 10 carbon atoms. In the case where substituted aromatic dicarboxylic acids are used, they will typically contain 1 to 4 substituents selected from halo, C₆-C₁₀ aryl, and C₁-C₄ alkoxy. Non-limiting axamples of aromatic dicarboxylic acids which may be used in the AAPE of our invention are terephthalic acid, isophthalic acid, salts of 5-sulfoisophthalic acid, and 2,6-naphthalenedicarboxylic acid. More preferably, the non-aromatic dicarboxylic acid will comprise adipic acid, the aromatic dicarboxylic add will comprise terephthalic acid, and the diol will comprise 1,4-butanediol.

Other possible compositions for the AAPE's of our invention are those prepared from the following diols and dicarboxylic acids (or polyester-forming equivalents thereof such as diesters) in the following mole percentages, based on 100 mole percent of a diacid component and 100 mole percent of a diol component:
(1) glutaric acid (30 to 75%); terephthalic acid (25 to 70%): 1,4-butanediol (90 to 100%); and modifying diol (0 to 10%):
(2) succinic acid (30 to 95%); terephthalic acid (5 to 70%); 1,4-butanediol (90 to 100%); and modifying diol (0 to 10%); and
(3) adipic acid (30 to 75%); terephthalic acid (25 to 70%); 1,4-butanediol (90 to 100%); and modifying diol (0 to 10%).

The modifying diol preferably is selected from 1,4-cyclohexanedimethanol, triethylene glycol, polyethylene glycol and neopentyl glycol. The most preferred AAPE's are linear, branched or chain extended copolyesters comprising 50 to 60 mole percent adipic acid residues, 40 to 50 mole percent terephthalic acid residues, and at least 95 mole percent 1,4-butanediol residues. Even more preferably, the adipic acid residues comprise 55 to 60 mole percent, the terephthalic acid residues comprise 40 to 45 mole percent, and the diol residues comprise 95 mole percent 1,4-butanediol residues. Such compositions are commercially available under the trademark EASTAR BIO® copolyester from Eastman Chemical Company, Kingsport, TN, and under the trademark ECOFLEX® from BASF Corporation.

Additional, specific examples of preferred AAPE's include a poly(tetxa-methylene glutarate-co-terephthalate) containing (a) 50 mole percent glutaric add residues, 50 mole percent terephthalic add residues, and 100 mole percent 1.4-butanediol residues, (b) 60 mole percent glutaric add residues, 40 mole percent terephthalic add residues; and 100 mole percent 1,4-butanediol residues or (c) 40 mole percent glutaric acid residues, 60 mole percent terephthalic acid residues, and 100 mole percent 1,4-butanediol residues; a poly(tatramethylene succinate-co-terephthalate) containing (a) 85 mole percent succinic acid residues, 15 mole percent terephthalic acid residues, and 100 mole percent 1,4-butanediol residues or (b) 70 mole percent succinic acid residues, 30 mole percent terephthalic add residues, and 100 mole percent 1,4-butanediol residues; a poly(ethylene succinate-co-terephthatate) containing 70 mole percent succinic add residues, 30 mole percent terephthalic add residues, and 100 mole percent ethylene glycol residues; and a poly (tetramethylene adipate-co-terephthalate) containing (a) 85 mole percent adipic add residues, 15 mole percent terephthalic acid residues, and 100 mole percent 1,4-butanediol residues; or (b) 55 mole percent adipic acid residues, 45 mole percent terephthalic acid residues, and 100 mole percent 1,4-butanediol residues.

The AAPE preferably comprises from 10 to 1,000 repeating units and preferably, from 15 to 600 repeating units. The AAPE may have an inherent viscosity of 0.4 to 2.0 dUg, or more preferably 0.7 to 1.6 dL/g, as measured at a temperature of 25°C using a concentration of 0.5 gram copolyester in 100 ml of a 60/40 by weight solution of phenol/tetrachloroethane.

The AAPE, optionally, may contain the residues of a branching agent. The mole percentage ranges for the branching agent are from 0 to 2 mole%, preferably 0.1 to 1 mole%, and most preferably 0.1 to 0.5 mole% based on the total moles of diacid or diol residues (depending on whether the branching agent contains carboxyl or hydroxyl groups). The branching agent preferably has a weight average molecular weight of 50 to 5000, more preferably 92 to 3000, and a functionality of 3 to 6. The branching agent, for example, may be the esterified residue of a polyol having 3 to 6 hydroxyl groups, a polycarboxylic add having 3 or 4 carboxyl groups (or ester-forming equivalent groups) or a hydroxy acid having a total of 3 to 6 hydroxyl and carboxyl groups. In addition, the AAPE may be branched by the addition of a peroxide during reactive extrusion.

Each segment of the water non-dispersible polymer may be different from others in fineness and may be arranged in any shaped or engineered cross-sectional geometry known to persons skilled in the art. For example, the sulfopolyester and a water-nondispersible polymer may be used to prepare a bicomponent fiber having an engineered geometry such as, for example, a sido-by-side, "islands-in-the-sea", segmented pie, other splitables, sheath/core, or other configurations known to persons skilled in the art. Other multicomponent configurations are also possible. Subsequent removal of a side, the "sea", or a portion of the "pie" can result in very fine fibers. The process of preparing bicomponent fibers also is well known to persons skilled in the art. In a bicomponent fiber, the sulfopolyester fibers of this invention may be present in amounts of 10 to 90 weight% and will generally be used in the sheath portion of sheath/core fibers. The other component may be from a wide range of other polymeric materials such as, for example, poly (ethylene) terephthalate, poly(butylene) terephthalate, poly(trimethylene) terephthalate, polylactides and the like as well as polyolefins, cellulose esters, and polyamides. Typically, when a water-insoluble or water-nondispersible polymer is used, the resulting bicomponent or multicomponent fiber is not completely water-dispersble. Side by side combinations with significant differences in thermal shrinkage can be utilized for the development of a spiral crimp. If crimping is desired, a saw tooth or stuffer box crimp is generally suitable for many applications. If the second polymer component is in the core of a sheath/core configuration, such a core optionally may be stabilized.

The sulfopolyesters are particularly useful for fibers having an "islands-in-the-sea" or "segmented pie" cross section as they only requires neutral or slightly acidic' (i.e., "soft" water) to disperse, as compared to the caustic-containing solutions that are sometimes required-to remove other water dispersible polymers from multicomponent fibers. One such multicomponent fiber comprises:
A) a water dispersible sulfopolyester having a glass transistion temperature (Tg) of at least 57°C, the sulfopolyester comprising:
   (i) 50 to 96 mole% of one or more residues of isophthalic acid orterephthalic acid, based on the total acid residues;
   (ii) 4 to 3 0 mole%, based on the total acid residues, of a residue of sodiosulfoisophthalic acid;
   (iii) one or more diol residues wherein at least 25 more%, based on the total diol residues, is a poly(ethylene glycol) having a structure

      H-(OCH₂-CH₂)ₙ-OH

      wherein n is an integer in the range of 2 to 500;
   (iv) 0 to 20 mole%, based on the total repeating units, of residues of a branching monomer having 3 or more functional groups wherein the functional groups are hydroxyl, carboxyl, or a combination thereof; and
B) a plurality of segments comprising one or more water-nondispersible polymers immiscible with the sulfopolyester,
   wherein the segments are substantially isolated from each other by the sulfopolyester intervening between the segments;
wherein the fiber has an islands-in-the-sea or segmented pie cross section and contains less than 10 weight percent of a pigment or filler, based on the total weight of the fiber.
The dicarboxylic acids, diols, sulfopolyester, sulfomonomers, branching monomers residues, and water non-dispersible polymers are as described previously. For multicomponent fibers, it is advantageous that sulfopolyester have a Tg of at least 57°C. The sulfopolyester may be a single sulfopolyester or a blend of one or more sulfopolyester polymers. Further examples of glass transition temperatures that may be exhibited by the sulfopolyester or sulfopolyester blends are at least 65°C, at least 70°C, at least 75°C, at least 85°C, and at least 90°C. For example, the sulfopolyester may comprise 75 to 96 mole% of one or more residues of isophthalic acid or terephthalic acid and 25 to 95 mole% of a residue of diethylene glycol. As described hereinabove, examples of the water-nondispersible polymers are polyolefins, polyesters, polyamides, polylactides, polycaprolactone, polycarbonate, polyurethane, and polyvinyl chloride. In addition, the water-nondispersible polymer may be biodegradable or biodisintegratable. For example, the water-nondispersible polymer may be an aliphatic-aromatic polyester as described previously.

Our novel multicomponent fiber may be prepared by any number of methods known to persons skilled in the art. The present invention thus provides a process for making a multicomponent fiber having a shaped cross section comprising: spinning a water dispersible sulfopolyester having a glass transistion temperature (Tg) of at least 57°C and one or more water-nondispersable polymers immiscible with the sulfopolyester into a fiber, the sulfopolyester comprising:
(i) residues of one or more dicarboxylic acids;
(ii) 4 to 40 mole%, based on the total repeating units, of residues of at least one sulfomonomer having 2 functional groups and one or more sulfonate groups attached to an aromatic or cycloaliphatic ring wherein the functional groups are hydroxyl, carboxyl, or a combination thereof;
(iii) one or more diol residues wherein at least 25 mole%, based on the total diol residues, is a poly(ethytene glycol) having a structure

   H-(OCH₂-CH₂)ₙ-OH

   wherein n is an integer in the range of 2 to 500; and
(iv) 0 to 25 mole%, based on the total repeating units, of residues of a branching monomer having 3 or more functional groups wherein the functional groups are hydroxyl, carboxyl, or a combination thereof;
wherein the fiber has a plurality of segments comprising the water-nondispersable polymers and the segments are substantially isolated from each other by the sulfopolyester intervening between the segments and the fiber contains less than 10 weight percent of a pigment or filler, based on the total weight of the fiber.
For example, the multicomponent fibermaybe prepared by melting the sulfopolyester and one or more water non-dispersible polymers in separate extruders and directing the individual polymer flows into one spinneret or extrusion die with a plurality of distribution flow paths such that the water non-dispersible polymer component form small segments or thin strands which are substantially isolated from each other by the intervening sulfopolyester. The cross section of such a fiber may be, for example, a segmented pie arrangement or an islauds-in-the-sea arrangement. In another example, the sulfopolyester and one or more water non-dispersible polymers are separately fed to the spinneret orifices and then extruded in sheath-core form in which the water non-dispersible polymer forms a "core" that is substantially enclosed by the sulfopolyester "sheath" polymer. In the case of such concentric fibers, the orifice supplying the "core" polymer is in the center of the spinning orifice outlet and flow conditions of core polymer fluid are strictly controlled to maintain the concentricity of both components when spinning Modifications in spinneret orifices enable different shapes of core and/or sheath to be obtained within the fiber cross-section. In yet another example, a multicomponent fiber having a side-by-side cross section or configuration may be produced by coextruding the water dispersible sulfopolyester and water non-dispersible polymer through orifices separately and converging the separate polymer streams at substantially the same speed to merge side-by-side as a combined stream below the face of the spinneret; or (2) by feeding the two polymer streams separately through orifices, which converge at the surface of the spinneret, at substantially the same speed to merge side-by-side as a combined stream at the surface of the spinneret. In both cases, the velocity of each polymer stream, at the point of merge, is determined by its metering pump speed, the number of orifices, and the size of the orifice.

The dicarboxylic acids, diols, sulfopolyester, sulfomonomers, branching monomers residues, and water non-dispersible polymers are as described previously. The sulfopolyester has a glass transistion temperature of at least 60°C. Further examples of glass transition temperatures that may be exhibited by the sulfopolyester or sulfopolyester blend are at least 65°C, at least 70°C, at least 75°C, at least 85°C, and at least 90°C. In one example, the sulfopolyester may comprise 50 to 96 mole% of one or more residues of isophthalic acid or terephthalic acid, based on the total acid residues; and 4 to 30 mole%, based on the total acid residues, of a residue of sodiosulfoisophthalic acid; and 0 to 20 mole%, based on the total repeating units, of residues of a branching monomer having 3 or more functional groups wherein the functional groups are hydroxyl, carboxyl, or a combination thereof. In another example, the sulfopolyester may comprise 75 to 96 mole% of one or more residues of isophthalic add or terephthalic add and 25 to 95 mole% of a residue of diethylene glycol. As described hereinabove, examples of the water-nondispersible polymers are polyolefins, polyesters, polyamides, polylactides, polycaprolactone, polycarbonate, polyurethane, and polyvinyl chloride. In addition, the water-nondispersible polymer may be biodegradable or biodisintegratable. For example, the water-nondispersible polymer may be an aliphatic-aromatic polyester as described previously. Examples of shaped cross sections include, but are not limited to, islands-in-the-sea, side-by-side, sheath-core, or segmented pie configurations.

Typically, upon exiting the spinneret, the fibers are quenched with a cross flow of air whereupon the fibers solidify. Various finishes and sizes may be applied to the fiber at this stage. The cooled fibers, typically, are subsequently drawn and wound up on a take up spool. Other additives may be incorporated in the finish in effective amounts like emulsifiers, antistatics, antimicrobials, antifoams, lubricants, thermostabilizers, UV stabilizers, and the like.

Optionally, the drawn fibers may be textured and wound-up to form a bulky continuous filament This one-step technique is known in the art as spin-draw-texturing. Other embodiments include flat filament (non-textured) yarns, or cut staple fiber, either crimped or uncrimped.

The sulfopolyester may be later removed by dissolving the interfacial layers or pie segments and leaving the smaller filaments or microdenier fibers of the water non-dispersible polymer(s). Our invention thus provides a process for making microdenier fibers comprising:
A. spinning a water dispersible sulfopolyester having a glass transistion temperature (Tg) of at least 57°C and one or more water-nondispersable polymers immiscible with the sulfopolyester into multicomponent fibers, the sulfopolyester comprising:
   (i) 50 to 96 mole% of one or more residues of isophthalic acid or terephthalic acid, based on the total acid residues;
   (ii) 4 to 30 mole%, based on the total acid residues, of a residue of sodiosulfoisophthalic acid;
   (iii)one or more diol residues wherein at least 25 mole%, based on the total diol residues, is a poly(ethylene glycol) having a structure

      H-(OCH₂-CH₂)ₙ-OH

      wherein n is an integer in the range of 2 to 500; and
   (iv)0 to 20 mole%, based on the total repeating units, of residues of a branching monomer having 3 or more functional groups wherein the functional groups are hydroxyl, carboxyl, or a combination thereof;
   wherein the fibers have a plurality of segments comprising the water-nondispersable polymers wherein the segments are substantially isolated from each other by the sulfopolyester intervening between the segments and the fibers contain less than 10 weight percent of a pigment or filler, based on the total weight of the fibers; and
B. contacting the multicomponent fibers with water to remove the sulfopolyester thereby forming microdenier fibers.
Typically, the multicomponent fiber is contacted with water at a temperature of 25°C to 100°C, preferably 50°C to 80°C for a time period of from 10 to 600 seconds whereby the sulfopolyester is dissipated or dissolved. After removal of the sulfopolyester, the remaining microfibers typically will have an average fineness of 1 d/f or less, typically, 0.5 d/f or less, or more typically, 0.1 d/f or less. Typical applications of these remaining microfibers include artificial leathers, suedes, wipes, and filter media. The ionic nature of sulfopolyesters also results in advantageously poor "solubility" in saline media, such as body fluids. Such properties are desirable in personal care products and cleaning wipes that are flushable or otherwise disposed in sanitary sewage systems. Selected sulfopolyesters have also been utilized as dispersing agents in dye baths and soil redeposition preventative agents during laundry cycles.

The instant invention also includes a fibrous article comprising the multicomponent fiber, or the microdenier fibers described hereinabove. The term fibrous article" is understood to mean any article having or resembling fibers. Non-limiting examples of fibrous articles include mulfilament fibers, yarns, cords, tapes, fabrics, melt blown webs, spunbonded webs, thermobonded webs, hydroentangled webs, nonwoven webs and fabrics, and combinations thereof; items having one or more layers of fibers, such as, for example, multilayer nonwovens, laminates, and composites from such fibers, gauzes, bandages, diapers, training pants, tampons, surgical gowns and masks, feminine napkins; and the like. Further, the fibrous articles may include replacement inserts for various personal hygiene and cleaning products. The fibrous article of the present invention may be bonded, laminated, attached to, or used in conjunction with other materials which may or may not be water-dispersible. The fibrous article, for example, a nonwoven fabric layer, may be bonded to a flexible plastic film or backing of a water-nondispersible material, such as polyethylene. Such an assembly, for example, could be used as one component of a disposable diaper. In addition, the fibrous article may result from overblowing fibers onto another substrate to form highly assorted combinations of engineered melt blown, spunbond, film., or membrane structures.

The fibrous articles of the instant invention include nonwoven fabrics and webs. A nonwoven fabric is defined as a fabric made directly from fibrous webs without weaving or knitting operations. For example, the multicomponent fiber of the present invention may be formed into a fabric by any known fabric forming process like knitting, weaving, needle punching, and hydroentangling. The resulting fabric or web may be converted into a microdenier fiber web by exerting sufficient force to cause the multicomponent fibers to split or by contacting the web with water to remove the sulfopolyester leaving the remaining microdenier fibers behind. Our invention thus provides a process for making a microdenier fiber web, comprising:
A. spinning a water dispersible sulfopolyester having a glass transistion temperature (Tg) of at least 57°C and one or more water-nondispersable polymers immiscible with the sulfopolyester into multicomponent fibers, the sulfpolyester comprising:
   (i) 50 to 96 mole% of one or more residues of isophthalic acid or terephthalic acid, based on the total acid residues;
   (ii) 4 to 30 mole%, based on the total acid residues, of a residue of sodiosulfoisophthalic acid;
   (iii)one or more diol residues wherein at least 25 mole%, based on the total diol residues, is a poly(ethylene glycol) having a structure

      H-(OCH₂-CH₂)ₙ-OH

      wherein n is an integer in the range of 2 to 500; and
   (iv)0 to 20 mole%, based on the total repeating units, of residues of a branching monomer having 3 or more functional groups wherein the functional groups are hydroxyl, carboxyl, or a combination thereof.
   wherein the multicomponent fibers have a plurality of segments comprising the water-nondispersable polymers
   wherein the segments are substantially isolated from each other by the sulfopolyester intervening between the segments; and the fiber contains less than 10 weight percent of a pigment or filler, based on the total weight of the fiber;
B. overlapping and collecting the multicomponent fibers of Step A to form a nonwoven web; and
C. contacting the nonwoven web with water to remove the sulfopolyester thereby forming a microdenier fiber web.

The nonwoven assembly is held together by 1) mechanical fiber cohesion and interlocking in a web or mat; 2) various techniques of fusing of fibers, including the use of binder fibers, utilizing the thermoplastic properties of certain polymers and polymer blends; 3) use of a binding resin such as starch, casein, a cellulose derivative, or a synthetic resin, such as an acrylic latex or urethane; 4) powder adhesive binders; or 5) combinations thereof. The fibers are often deposited in a random manner, although orientation in one direction is possible, followed by bonding using one of the methods described above.

The fibrous articles of our invention further also may comprise one or more layers of multicomponent fibers, or microdenier fibers. The fiber layers may be one or more nonwoven fabric layers, a layer of loosely bound overlapping fibers, or a combination thereof. In addition, the fibrous articles may include personal and health care products such as, but not limited to, child care products, such as infant diapers; child training pants; adult care products, such as adult diapers and adult incontinence pads; feminine care products, such as feminine napkins, panty liners, and tampons; wipes; fiber-containing deaning products; medical and surgical care products, such as medical wipes, tissues, gauzes, examination bed coverings, surgical masks, gowns, bandages, and wound dressings; fabrics; elastomeric yarns, wipes, tapes, other protective barriers, and packaging material. The fibrous articles may be used to absorb liquids or may be pre-moistened with various liquid compositions and used to deliver these compositions to a surface. Non-limiting examples of liquid compositions include detergents; wetting agents; deaning agents; skin care products, such as cosmetics, ointments, medications, emollients, and fragrances. The fibrous articles also may include various powders and particulates to improve absorbency or as delivery vehicles. Examples of powders and particulates include, but are not limited to, talc, starches, various water absorbent, water-dispersible, or water swellable polymers, such as super absorbent polymers, sulfopolyesters, and poly(vinylalcohols), silica, pigments, and microcapsules. Additives may also be present, but are not required, as needed for specific applications. Examples of additives include, but are not limited to, oxidative stabilizers, UV absorbers, colorants, pigments, opacifiers (delustrants), optical brighteners, fillers, nucleating agents, plasticizers, viscosity modifiers, surface modifiers, antimicrobials, disinfectants, cold now inhibitors, branching agents, and catalysts.

In addition to being water-dispersible, the fibrous articles described above may be flushable. The term "flushable" as used herein means capable of being flushed in a conventional toilet, and being introduced into a municipal sewage or residential septic system, without causing an obstruction or blockage in the toilet or sewage system.

The fibrous article may further comprise a water-dispersible film comprising a second water-dispersible polymer. The second water-dispersible polymer maybe the same as or different from the previously described water-dispersible polymers used in the fibers and fibrous articles of the present invention. In one embodiment, for example, the second water-dispersible polymer may be an additional sulfopolyester which, in turn, comprises: (i) 50 to 96 mole% of one or more residues of isophthalic acid or terephthalic acid, based on the total acid residues; (ii) 4 to 30 mole%, based on the total acid residues, of a residue of sodiosulfoisophthalic acid; (iii) one or more diol residues wherein at least 15 mole%, based on the total diol residues, is a poly(ethylene glycol) having a structure

H-(OCH₂-CH₂)ₙ-OH

wherein n is an integer in the range of 2 to 500; (iv) 0 to 20 mole%, based on the total repeating units, of residues of a branching monomer having 3 or more functional groups
wherein the functional groups are hydroxyl, carboxyl, or a combination thereof. The additional sulfopolyester may be blended with one or more supplemental polymers, as described hereinabove, to modify the properties of the resulting fibrous article. The supplemental polymer may or may not be water-dispersible depending on the application. The supplemental polymer may be miscible or immiscible with the additional sulfopolyester.

The additional sulfopolyester may contain other concentrations of isophthalic acid residues, for example, 60 to 95 mole%, and 75 to 95 mole%. Further examples of isophthalic acid residue concentrations ranges are 70 to 85 mole%, 85 to 95 mole% and 90 to 95 mole%. The additional sulfopolyester also may comprise 25 to 95 mole% of the residues of diethylene glycol. Further examples of diethylene glycol residue concentration ranges include 50 to 95 mole%, 70 to 95 mole%, and 75 to 95 mole%. The additional sulfopolyester also may include the residues of ethylene glycol and/or 1,4-cyclohexanedimethanol. Typical concentration ranges of CHDM residues are 10 to 75 mole%, 25 to 65 mole%, and 40 to 60 mole%. Typical concentration ranges of ethylene glycol residues are are 10 to 75 mole%, 25 to 65 mole%, and 40 to 60 mole%. In another embodiment, the additional sulfopolyester comprises is 75 to 96 mole% of the residues of isophthalic acid and 25 to 95 mole% of the residues of diethylene glycol.

According to the invention, the sulfopolyester film component of the fibrous article may be produced as a monolayer or multilayer film. The monolayer film may be produced by conventional casting techniques. The multilayered films may be produced by conventional lamination methods or the like. The film may be of any convenient thickness, but total thickness will normally be between 2 and 50 mil.

The film-containing fibrous articles may include one or more layers of water-dispersible fibers as described above. The fiber layers may be one or more nonwoven fabric layers, a layer of loosely bound overlapping fibers, or a combination thereof. In addition, the film-containing fibrous articles may include personal and health care products as described hereinabove.

As described previously, the fibrous articles also may include various powders and particulates to improve absorbency or as delivery vehicles. Thus, in one embodiment, our fibrous article comprises a powder comprising a third water-dispersible polymer that may be the same as or different from the water-dispersible polymer components described previously herein. Other examples of powders and particulates include, but are not limited to, talc, starches, various water absorbent, water-dispersible, or water swellable polymers, such as poly(acrylonitiles), sulfopolyesters, and poly (vinyl alcohols), silica, pigments, and microcapsules.

Our novel fiber and fibrous articles have many possible uses in addition to the applications described above. One novel application involves the melt blowing a film or nonwoven fabric onto flat, curved, or shaped surfaces to provide a protective layer. One such layer might provide surface protection to durable equipment during shipping. At the destination, before putting the equipment into service, the outer layers of sulfopolyester could be washed off. A further embodiment of this general application concept could involve articles of personal protection to provide temporary barrier layers for some reusable or limited use garments or coverings. For the military, activated carbon and chemical absorbers could be sprayed onto the attenuating filament pattern just prior to the collector to allow the melt blown matrix to anchor these entities on the exposed surface. The chemical absorbers can even be changed in the forward operations area as the threat evolves by melt blowing on another layer.

A major advantage inherent to sulfopolyesters is the facile ability to remove or recover the polymer from aqueous dispersions via flocculation or precipitation by adding ionic moieties (i.e., salts). Other methods, such as pH adjustment, adding nonsolvents, freezing, and so forth may also be employed. Therefore, fibrous articles, such as outer wear protective garments, after successful protective barrier use and even if the polymer is rendered as hazardous waste, can potentially be handled safely at much lower volumes for disposal using accepted protocols, such as incineration.

Undissolved or dried sulfopolyesters are known to form strong adhesive bonds to a wide array of substrates, including, but not limited to fluff pulp, cotton, acrylics, rayon, lyocell, PLA (polylactides), cellulose acetate, cellulose acetate propionate, poly(ethylene) terephthalate, poly(butylene) terephthalate, poly(bimethylene) terephthalate, poly (cyclohexylene) terephthalate, copolyesters, polyamides (nylons), stainless steel, aluminum, treated polyolefins, PAN (polyacrylonitriles), and polycarbonates. Thus, our nonwoven fabrics may be used as laminating adhesives or binders that may be bonded by known techniques, such as thermal, radio frequency (RF), microwave, and ultrasonic methods. Adaptation of sulfopolyesters to enable RF activation is disclosed in a number of recent patents. Thus, our novel nonwoven fabrics may have dual or even multifunctionality in addition to adhesive properties. For example, a disposable baby diaper could be obtained where a nonwoven of the present invention serves as both an water-responsive adhesive as well as a fluid managing component of the final assembly.

The sulfomonomer may be a dicarboxylic acid or ester thereof containing a sulfonate group, a diol containing a sulfonate group, or a hydroxy acid containing a sulfonate group. Additional examples of concentration ranges for the sulfomonomer residues are 4 to 25 mole%, 4 to 20 mole%, 4 to 15 mole%, and 4 to 10 mole%, based on the total repeating units. The cation of the sulfonate salt may be a metal ion such as Li⁺, Na⁺, K⁺, Mg⁺⁺, Ca⁺⁺, Ni⁺⁺, Fe⁺⁺, and the like. Alternatively, the cation of the sulfonate salt may be non-metallic such as a nitrogenous base as described previously. Examples examples of sulfomonomer residues which may be used in the process of the present invention are the metal sulfonate salt of sulfophthalic acid, sulfoterephthalic acid, sulfoisophthalic acid, or combinations thereof. Another example of sulfomonomer which may be used is 5-sodiosutfoisophthalic acid or esters thereof. If the sulfomonomer residue is from 5-sodiosulfoisophthalic acid, typical sulfomonomer concentration ranges are 4 to 35 mole%, 8 to 30 mole %, and 10 to 25 mole %, based on the total acid residues.

The sulfopolyester includes one or more diol residues which may include aliphatic, cycloaliphatic, and aralkyl glycols. The cycloaliphatic diols, for example, 1.3- and 1,4-cyclohexanedimethanol, may be present as their pure cis or transisomers or as a mixture of cis and *trans* isomers. Non-limiting examples of lower molecularweight polyethylene glycols, e.g., wherein n is from 2 to 6, are diethylene glycol, triethylene glycol, and tetraethylene glycol. Of these lower molecular weight glycols, diethylene and triethylene glycol are most preferred. The sulfopolyester may optionally include a branching monomer. Examples of branching monomers are as described hereinabove. Further examples of branching monomer concentration ranges are from 0 to 20 mole% and from 0 to 10 mole%.

The water-dispersible fibers are prepared by a melt blowing process. The polymer is melted in an extruder and forced through a die. The extrudate exiting the die is rapidly attenuated to ultrafine diameters by hot, high velocity air. The orientation, rate of cooling, glass transition temperature (T_{g}), and rate of crystallization of the fiber are important because they affect the viscosity and processing properties of the polymer during attenuation. The filament is collected on a renewable surface, such as a moving belt, cylindrical drum, rotating mandrel, and so forth. Predrying of pellets (if needed), extruder zone temperature, melt temperature, screw design, throughput rate, air temperature, air flow (velocity), die air gap and set back, nose tip hole size, die temperature, die-to-collector (DCP) distance, quenching environment, collector speed, and post treatments are all factors that influence product characteristics such as filament diameters, basis weight, web thickness, pore size, softness, and shrinkage. The high velocity air also may be used to move the filaments in a somewhat random fashion that results in extensive interlacing. If a moving belt is passed under the die, a nonwoven fabric can be produced by a combination of over-lapping laydown, mechanical cohesiveness, and thermal bonding of the filaments. Overblowing onto another substrate, such as a spunbond or backing layer, is also possible. If the filaments are taken up on an rotating mandrel, a cylindrical product is formed. A water-dispersible fiber lay-down can also be prepared by the spunbond process. The invention is further illustrated by the following examples.

### EXAMPLES

All pellet samples were predried under vacuum at room temperature for at least 12 hours. The dispersion times shown in Table 3 are for either complete dispersion or dissolution of the nonwoven fabric samples. The abbreviation "CE" used in Tables 2 and 3 mean "comparative example".

### Example 1 (illustrative example)

A sulfopolyester containing 76 mole%, isophthalic acid, 24 mole% of sodiosulfoisophthalic acid, 76 mole% diethylene glycol, and 24 mole% 1,4-cydohexanedimethanol with an Ih.V. of 0.29 and a Tg of 48°C was meltblown through a nominal 6-inch die (30 holes/inch in the nosepiece) onto a cylindrical collector using the conditions shown in Table 1. Interleafing paper was not required. A soft, handleable, flexible web was obtained that did not block during the roll winding operation. Physical properties are provided in Table 2. A small piece (1" x 3") of the nonwoven fabric was easily dispersed in both room temperature (RT) and 50°C water with slight agitation as shown by data in Table 3.

**Table 1 - Melt Blowing Conditions**

| Operating Condition | Typical Value |
|---|---|
| *Die Configuration* | |
| Die tip hole diameter | 0.0185 inches |
| Number of holes | 120 |
| Air gap | 0.060 inches |
| Set back | 0.060 inches |

| *Extruder Barrel* | *Temperatures (⁰F)* |
|---|---|
| Zone 1 | 350 |
| Zone 2 | 510 |
| Zone 3 | 510 |

| *Die Temperatures (⁰F)* | |
|---|---|
| Zone 4 | 510 |
| Zone 5 | 510 |
| Zone 6 | 510 |
| Zone 7 | 510 |
| Zone 8 | 510 |

| *Air Temperatures (⁰F)* | |
|---|---|
| Furnace exit 1 | 350 |
| Furnace exit 2 | 700 |
| Furnace exit 3 | 700 |
| Die | 530-546 |

| *Extrusion Conditions* | |
|---|---|
| Air pressure | 3.0 psi |
| Melt pressure after pump | 99-113 psi |

| *Take Up Conditions* | |
|---|---|
| Throughput | 0.3 g/hole/min |
| | 0.5 g/hote/min |
| Basis weight | 36 g/m² |
| Collector speed | 20 ft/min |
| Collector distance | 12 inches |

**Table 2 - Physical Properties of Nonwovens**

| Example | Filament Diameter (µm) | | | IhV (before/after) | Tg/Tm (°C) (sulfopoly. / PP) |
|---|---|---|---|---|---|
| | Minimum | Maximum | Average | | |
| 1 | 5 | 18 | 8.7 | 0.29/0.26 | 39/ not applicable |
| 2 | 3 | 11 | 7.7 | 0.40/0.34 | 36/notapplicable |
| CE 1 | 2 | 20 | 8 | Not measured | 36/163 |
| CE 2 | 4 | 10 | 7 | Not measured | 36/164 |
| CE 3 | 4 | 11 | 6 | Not measured | 35/161 |

**Table 3 - Dispersbility of Nonwovens**

| Example | Water Temperature (°C) | Initial Disintegration (minutes) | Significant Disintegration (minutes) | Complete Dispersion (minutes) |
|---|---|---|---|---|
| 1 | 23 | <0.25 | 1 | 2 |
| | 50 | <0.17 | 0.5 | 1 |
| 2 | 23 | 8 | 14 | 19 |
| | 50 | <0.5 | 5 | 8 |
| | 80 | <0.5 | 2 | 5 |
| CE 1 | 23 | 0.5 | >15 | No dispersion of PP |
| | 50 | 0.5 | >15 | No dispersion of PP |
| CE 2 | 23 | 0.5 | >15 | No dispersion of PP |
| | 50 | 0.5 | >15 | No dispersion of PP |
| CE 3 | 23 | <0.5 | 6 | No dispersion of PP |
| | 50 | <0.5 | 4 | No dispersion of PP |

### Example 2 (illustrative example)

A sulfopolyester containing 89 mole%, isophthalic acid, 11 mole% of sodiosulfoisophthalic acid, 72 mole% diethylene glycol, and 28 mole% ethylene glycol with an lh.V. of 0.4 and a Tg of 35°C was meltblown through a 6-inch die using conditions similar to those in Table 1. A soft, handleable, flexibly web was obtained that did not block during a roll winding operation. Physical properties are provided in Table 2. A small piece (1"x 2") of the nonwoven fabric was easily and completely dispersed at 50°C and 80°C; at RT (23°C), the fabric required a longer period of time for complete dispersion as shown by the data in Table 3.

It was found that the compositions in Examples 1 and 2 can be overblown onto other nonwoven substrates. It is also possible to condense and wrap shaped or contoured forms that are used instead of conventional web collectors. Thus, it Is possible to obtain circular "roving" or plug forms of the webs.

### Comparative Examples 1-3

Pellets of a sulfopolyester containing 89 mole%, isophthalic acid, 11 mole% of sodiosulfoisophthalic acid, 72 mole% diethylene glycol, and 28 mole% ethylene glycol with an lh. V. of 0.4 and a Tg of 35°C were combined with polypropylene (Basell PF 008) pellets in bicomponent ratios (by wt%) of :
75 PP : 25 sulfopolyester (Example 3)
50 PP : 50 sulfopolyester (Example 4)
25 PP : 75 sulfopolyester (Example 5)

The PP had a MFR (melt flow rate) of 800. A melt blowing operation was performed on a line equipped with a 24-inch wide die to yield handleable, soft, flexible, but nonblocking webs with the physical properties provided in Table 2. Small pieces (1" x 4") of nonwoven fabric readily disintegrated as reported in Table 3. None of the fibers, however, were completely water-dispersible because of the insoluble polypropylene component

### Example 3 (illustrative example)

A circular piece (4" diameter) of the nonwoven produced in Example 2 was used as an adhesive layer between two sheets of cotton fabric. A Hannifin melt press was used to fuse the two sheets of cotton together by applying a pressure 35 psig at 200°C for 30 seconds. The resultant assembly exhibited exceptionally strong bond strength. The cotton substrate shredded before adhesive or bond failure. Similar results have also been obtained with other cellulosics and with PET polyester substrates. Strong bonds were also produced by ultrasonic bonding techniques.

### Comparative Example 4

A PP (Exxon 3356G) with a 1200 MFR was melt blown using a 24" die to yield a flexible nonwoven fabric that did not block and was easily unwound from a roll. Small pieces (1"x 4") did not show any response (i.e., no disintegration or loss in basis weight) to water when immersed in water at RT or 50°C for 15 minutes.

### Example 4 (illustrative example)

Unicomponent fibers of a sulfopolyester containing 82 mole% isophthalic acid. 18 mole% of sodiosulfoisophthalic acid, 54 mole% diethylene glycol, and 46 mole% 1,4-cydohexanedinaethanol with a Tg of 55°C were melt spun at melt temperatures of 245°C (473°F) on a lab staple spinning line. As-spun denier was approximately 8 d/f. Some blocking was encountered on the take-up tubes, but the 10-filament strand readily dissolved within 10-19 seconds in unagitated, demineralized water at 82°C and a pH between 5 and 6.

### Example 5 (in accordance with the present invention together with Example 6)

Unicomponent fibers obtained from a blend (75:25) of a sulfopolyester containing 82 mole% isophthalic acid, 18 mole% of sodiosulfoisophthalic acid, 54 mole% diethylene glycol, and 46 mole% 1,4-cydohexanedimethanol (Tg of 55°C) and a sulfopolyester containing 91 mole% isophthalic acid, 9 mole% of sodiosulfoisophthalic acid, 25 mole% diethylene glycol, and 75 mole% 1,4-cyclohexanedimethanol (Tg of 65°C), respectively, were melt spun on a lab staple spinning line. The blend has a Tg of 57°C as calculated by taking a weighted average of the Tg's of the component sulfopolyesters. The 10-filament strands did not show any blocking on the take-up tubes, but readily dissolved within 20 - 43 seconds in unagitated, demineralized water at 82° C and a pH between 5 and 6.

### Example 6 (in accordance with the present invention)

The blend described in Example 5 was co-spun with PET to yield bicomponent islands-in-the-sea fibers. A configuration was obtained where the sulfopolyester "sea" is 20 wt% of the fiber containing 80 wt% of PET "islands". The spun yam elongation was 190% immediately after spinning. Blocking was not encountered as the yam was satisfactorily unwound from the bobbins and processed a week after spinning. In a subsequent operation, the "sea" was dissolved by passing the yam through an 88°C soft water bath leaving only fine PET filaments.

### Example 7 (in accordance with the present invention)

This prophetic example illustrates the possible application of the multicomponent and microdenier fibers of the present invention to the preparation of specialty papers. The blend described in Example 5 is co-spun with PET to yield bicomponent islands-in-the-sea fibers. The fiber contains approximately 35 wt% sulfopolyester "sea" component and approximately 65 wt% of PET "islands". The uncrimped fiber is cut to 1/8 inch lengths. In simulated papermaking, these short-cut bicomponent fibers are added to the refining operation. The sulfopolyester "sea" is removed in the agitated, aqueous slurry thereby releasing the microdenier PET fibers into the mix. At comparable weights, the microdenier PET fibers ("islands") are more effective to increase paper tensile strength than the addition of coarse PET fibers.

## Claims

1. A multicomponent fiber having a shaped cross section, comprising:
(A) a water dispersible sulfopolyester having a glass transistion temperature (Tg) of at least 57°C, said sulfopolyester comprising:
(i) residues of one or more dicarboxylic acids;
(ii) 4 to 40 mole%, based on the total repeating units, of residues of at least one sulfomonomer having 2 functional groups and one or more sulfonate groups attached to an aromatic or cycloaliphatic ring wherein said functional groups are hydroxyl, carboxyl, or a combination thereof;
(iii) one or more diol residues wherein at least 25 mole%, based on the total diol residues, is a poly(ethylene glycol) having a structure
H-(OCH₂-CH₂)ₙ-OH
wherein n is an integer in the range of 2 to 500; and
(iv) 0 to 25 mole%, based on the total repeating units, of residues of a branching monomer having 3 or more functional groups wherein said functional groups are hydroxyl, carboxyl, or a combination thereof; and
(B) a plurality of segments comprising one or more water-nondispersable polymers immiscible with said sulfopolyester,
wherein said segments are substantially isolated from each other by said sulfopolyester intervening between said segments;
wherein said fiber contains less than 10 weight percent of a pigment or filler, based on the total weight of said fiber.

2. The fiber according to claim 1 in which said dicarboxylic acids are selected from aliphatic diacids, cycloaliphatic dicarboxylic acids, aromatic dicarboxylic acids, and combinations thereof.

3. The fiber according to claim 2 in which said dicarboxylic acids are selected from succinic, glutaric, adipic, azelaic, sebacic, fumaric, maleic, itaconic, 1,3-cyclohexane dicarboxylic, 1,4-cyclohexanedicarboxylic, diglycolic, 2,5-norbomanedicarboxylic, phthalic, terephthalic, 1,4-naphthalenedicarboxylic, 2,5-naphthalenedicarboxylic, 2,6-naphthalenedicarboxylic, 2,7-naphthalenedicarboxylic, diphenic, 4,4'-oxydibenzoic, 4,4'-sulfonyldibenzoic, isophthalic, and combinations thereof.

4. The fiber according to any one of claims 1 to 3 in which said sulfomonomer is a metal sulfonate salt of a sulfophthalic acid, sulfoterephthalic acid, sulfoisophthalic acid, or combinations thereof.

5. The fiber according to any one of claims 1 to 4 in which said diol residues are selected from ethylene glycol, diethylene glycol, triethylene glycol, poly(ethylene) glycols, 1,3-propanediol, 2,4-dimethyl-2-ethylhexane-1,3-diol, 2,2-dimethyl-1,3-propanediol, 2-ethyl-2-butyl-1,3-propanediol, 2-ethyl-2-isobutyl-1,3-propanediol, 1,3-butanediol, 1,4-butanediol, 1,5-pentanediol, 1,6-hexanediol, 2,2,4-trimethyl-1,6-hexanediol, thiodiethanol, 1,2-cyclohexanedimethanol, 1,3-cyclohexanedimethanol, 1,4-cyclohexanedimethanol, 2,2,4,4-tetramethyl-1,3-cyclobutanediol, p-xylylenediol, and combinations thereof.

6. The fiber according to any one of claims 1 to 5 in which said branching monomer is 1,1,1-trimethylol propane, 1,1,1-trimethylolethane, glycerin, pentaerythritol, erythritol, threitol, dipentaerythritol, sorbitol, trimellitic anhydride, pyromellitic dianhydride, dimethylol propionic acid, or combinations thereof.

7. The fiber according to any one of claims 1 to 6 in which said sulfopolyester comprises 50 to 96 mole% of one or more residues of isophthalic acid or terephthalic acid, based on the total acid residues; 4 to 30 mole%, based on the total acid residues, of a residue of sodiosulfoisophthalic acid; and 0 to 20 mole%, based on the total repeating units, of residues of a branching monomer having 3 or more functional groups wherein said functional groups are hydroxyl, carboxyl, or a combination thereof; and wherein said shaped cross section is an islands-in-the-sea or segmented pie.

8. The fiber according to any one of claims 1 to 7 wherein said water-nondispersible polymers are selected from polyolefins, polyesters, polyamides, polylactides, polycaprolactone, polycarbonate, polyurethane, polyvinyl chloride, and combinations thereof.

9. The fiber according to any one of claims 1 to 8 wherein said water-nondispersible polymer is biodistintegratable as determined by DIN Standard 54900 or biodegradable as determined by ASTM Standard Method, D6340-98.

10. A fibrous article comprising the fiber of any one of claims 1 to 9.

11. The fibrous article of claim 10 in which said article is selected from a yarn, fabric, melt blown web, spunbonded web, thermobonded web, hydroentangled web, nonwoven fabric, and combinations thereof.

12. A process for making a multicomponent fiber having a shaped cross section comprising:
(A) spinning a water dispersible sulfopolyester having a glass transistion temperature (Tg) of at least 57°C and one or more water-nondispersable polymers immiscible with said sulfopolyester into a fiber, said sulfopolyester comprising:
(i) residues of one or more dicarboxylic acids;
(ii) 4 to 40 mole%, based on the total repeating units, of residues of at least one sulfomonomer having 2 functional groups and one or more sulfonate groups attached to an aromatic or cycloaliphatic ring wherein said functional groups are hydroxyl, carboxyl, or a combination thereof,
(iii) one or more diol residues wherein at least 25 mole%, based on the total diol residues, is a poly(ethylene glycol) having a structure
H-(OCH₂CH₂)ₙ-OH
wherein n is an integer in the range of 2 to 500; and
(iv) 0 to 25 mole%, based on the total repeating units, of residues of a branching monomer having 3 or more functional groups wherein said functional groups are hydroxyl, carboxyl, or a combination thereof;
wherein said fiber has a plurality of segments comprising said water-nondispersable polymers and said segments are substantially isolated from each other by said sulfopolyester intervening between said segments
and wherein said fiber contains less than 10 weight percent of a pigment or filler, based on the total weight of said fiber.

13. The process according to claim 12 wherein said sulfopolyester comprises 50 to 96 mole% of one or more residues of isophthalic acid or terephthalic acid, based on the total acid residues; 4 to 30 mole%, based on the total acid residues, of a residue of sodiosulfoisophthalic acid; and 0 to 20 mole%, based on the total repeating units, of residues of a branching monomer having 3 or more functional groups wherein said functional groups are hydroxyl, carboxyl, or a combination thereof; and wherein said shaped cross section is an islands-in-the-sea or segmented pie.

14. A process for making microdenier fibers comprising:
(A) spinning the multicomponent fibers of claim 12 comprising the water dispersible sulfopolyester of claim 13; and
(B) contacting the multicomponent fibers from step (A) with water to remove said sulfopolyester thereby forming microdenier fibers comprising said water non-dispersable polymers.

15. A fibrous article comprising the microdenier fibers of claim 14.

16. A process for making a microdenier fiber web, comprising:
(A) spinning the multicomponent fiber of claim 12 comprising water dispersible sulfopolyester of claim 13;
(B) overlapping and collecting the multicomponent fibers of step (A) to form a nonwoven web; and
(C) contacting said nonwoven web with water to remove said sulfopolyester thereby forming a microdenier fiber web.

## Patentansprüche

1. Mehrkomponentenfaser mit einem geformten Querschnitt umfassend:
(A) einen wasserdispergierbaren Sulfopolyester mit einer Glasübergangstemperatur (Tg) von mindestens 57 °C, wobei besagter Sulfopolyester umfasst:
(i) Reste einer oder mehrerer Dicarbonsäuren;
(ii) 4 bis 40 Mol-%, bezogen auf die gesamten sich wiederholenden Einheiten, an Resten mindestens eines Sulfomonomers mit zwei funktionellen Gruppen und einer oder mehreren Sulfonatgruppen, die an einen aromatischen oder cycloaliphatischen Ring gebunden sind, wobei besagte funktionelle Gruppen Hydroxyl, Carboxyl, oder eine Kombination davon sind;
(iii) einen oder mehrere Diolreste, wobei mindestens 25 Mol-%, bezogen auf die gesamten Diolreste, ein Polyethylenglykol ist mit einer Struktur
H-(OCH₂-CH₂)ₙ-OH
wobei n eine ganze Zahl im Bereich von 2 bis 500 ist; und
(iv) 0 bis 25 Mol-%, bezogen auf die gesamten sich wiederholenden Einheiten, an Resten eines Verzweigungsmonomers mit drei oder mehr funktionellen Gruppen, wobei besagte funktionelle Gruppen Hydroxyl, Carboxyl, oder eine Kombination davon sind; und
(B) eine Vielzahl von Segmenten umfassend ein oder mehrere Polymere, die in Wasser nicht dispergierbar sind, welche nicht mischbar mit besagtem Sulfopolyester sind, wobei besagte Segmente im Wesentlichen voneinander durch besagten Sulfopolyester isoliert sind, der zwischen besagten Segmenten liegt;
wobei besagte Faser weniger als 10 Gewichts-% eines Pigments oder Füllstoffs enthält, bezogen auf das Gesamtgewicht der besagten Faser.

2. Faser nach Anspruch 1, wobei besagte Dicarbonsäuren ausgewählt sind aus aliphatischen Disäuren, cycloaliphatischen Dicarbonsäuren, aromatischen Dicarbonsäuren, und Kombinationen davon.

3. Faser nach Anspruch 2, wobei besagte Dicarbonsäuren ausgewählt sind aus Bernsteinsäure, Glutarsäure, Adipinsäure, Azelainsäure, Sebacinsäure, Fumarsäure, Maleinsäure, Itaconsäure, 1,3-Cyclohexandicarbonsäure, 1,4-Cyclohexandicarbonsäure, Diglycolsäure, 2,5-Norbornandicarbonsäure, Phthalsäure, Terephthalsäure, 1,4-Naphthalindicarbonsäure, 2,5-Naphthalindicarbonsäure, 2,6-Naphthalindicarbonsäure, 2,7-Naphthalindicarbonsäure, Diphensäure, 4,4'-Oxydibenzoesäure, 4,4'-Sulfonyldibenzoesäure, Isophthalsäure, und Kombinationen davon.

4. Faser nach einem der Ansprüche 1 bis 3, wobei besagtes Sulfomonomer ein Metallsulfonatsalz einer Sulfophthalsäure, Sulfoterephthalsäure, Sulfoisophthalsäure, oder Kombinationen davon ist.

5. Faser nach einem der Ansprüche 1 bis 4, wobei besagte Diolreste ausgewählt sind aus Ethylenglykol, Diethylenglykol, Triethylenglykol, Polyethylenglykolen, 1,3-Propandiol, 2,4-Dimethyl-2-ethylhexan-1,3-diol, 2,2-Dimethyl-1,3-propandiol, 2-Ethyl-2-butyl-1,3-propandiol, 2-Ethyl-2-isobutyl-1,3-propandiol, 1,3-Butandiol, 1,4-Butandiol, 1,5-Pentandiol, 1,6-Hexandiol, 2,2,4-Trimethyl-1,6-hexandiol, Thiodiethanol, 1,2-Cyclohexandimethanol, 1,3-Cyclohexandimethanol, 1,4-Cyclohexandimethanol, 2,2,4,4-Tetramethyl-1,3-cyclobutandiol, p-Xyloldiol, und Kombinationen davon.

6. Faser nach einem der Ansprüche 1 bis 5, wobei besagtes Verzweigungsmonomer 1,1,1-Trimethylolpropan, 1,1,1-Trimethylolethan, Glycerin, Pentaerythrit, Erythrit, Threit, Dipentaerythrit, Sorbit, Trimellitsäureanhydrid, Pyromellitsäuredianhydrid, Dimethylpropionsäure, oder Kombinationen davon ist.

7. Faser nach einem der Ansprüche 1 bis 6, wobei besagter Sulfopolyester umfasst: 50 bis 96 Mol-% eines oder mehrerer Reste von Isophthalsäure oder Terephthalsäure, bezogen auf die gesamten Säurereste; 4 bis 30 Mol-%, bezogen auf die gesamten Säurereste, eines Restes der Natriumsulfoisophthalsäure; und 0 bis 20 Mol-%, bezogen auf die gesamten sich wiederholenden Einheiten, von Resten eines Verzweigungsmonomers mit drei oder mehr funktionellen Gruppen, wobei besagte funktionelle Gruppen Hydroxyl, Carboxyl, oder eine Kombination davon sind; und wobei besagter Querschnitt die Form einer Insel (Island-in-the-Sea-Querschnitt) oder eines Tortenstücks (Segmented-Pie-Querschnitt) hat.

8. Faser nach einem der Ansprüche 1 bis 7, wobei besagte Polymere, welche in Wasser nicht dispergierbar sind, ausgewählt sind aus Polyolefinen, Polyestern, Polyamiden, Polylactiden, Polycaprolacton, Polycarbonat, Polyurethan, Polyvinylchlorid, und Kombinationen davon.

9. Faser nach einem der Ansprüche 1 bis 8, wobei besagtes Polymer, welches in Wasser nicht dispergierbar ist, biozersetzbar ist wie bestimmt nach DIN Standard 54900, oder bioabbaubar ist wie bestimmt nach ASTM Standardmethode D6340-98.

10. Faserförmiger Gegenstand umfassend die Faser nach einem der Ansprüche 1 bis 9.

11. Faserförmiger Gegenstand nach Anspruch 10, wobei besagter Gegenstand ausgewählt ist aus: Garn, Gewebe, schmelzgeblasenes Gewebe, Spinnvlies, thermogebundenes Gewebe, hydroverschränktes Gewebe, Faservlies, und Kombinationen davon.

12. Verfahren zum Herstellen einer Mehrkomponentenfaser mit einem geformten Querschnitt umfassend:
(A) Spinnen eines wasserdispergierbaren Sulfopolyesters mit einer Glasübergangstemperatur (Tg) von mindestens 57 °C und eines oder mehrerer Polymeren, welche in Wasser nicht dispergierbar sind, und welche mit besagtem Sulfopolyester nicht mischbar sind, in eine Faser, wobei besagter Sulfopolyester umfasst:
(i) Reste einer oder mehrerer Dicarbonsäuren;
(ii) 4 bis 40 Mol-%, bezogen auf die gesamten sich wiederholenden Einheiten, an Resten mindestens eines Sulfomonomers mit zwei funktionellen Gruppen und einer oder mehreren Sulfonatgruppen, die an einen aromatischen oder cycloaliphatischen Ring gebunden sind, wobei besagte funktionelle Gruppen Hydroxyl, Carboxyl, oder eine Kombination davon sind;
(iii) ein oder mehrere Diolreste, wobei mindestens 25 Mol-%, bezogen auf die gesamten Diolreste, ein Polyethylenglykol ist mit einer Struktur
H-(OCH₂-CH₂)ₙ-OH
wobei n eine ganze Zahl im Bereich von 2 bis 500 ist; und
(iv) 0 bis 25 Mol-%, bezogen auf die gesamten sich wiederholenden Einheiten, an Resten eines Verzweigungsmonomers mit drei oder mehr funktionellen Gruppen, wobei besagte funktionelle Gruppen Hydroxyl, Carboxyl, oder eine Kombination davon sind;
wobei besagte Faser eine Vielzahl von Segmenten aufweist, welche besagte Polymere, welche in Wasser nicht dispergierbar sind, umfassen, und wobei besagte Segmente im Wesentlichen voneinander durch besagten Sulfopolyester isoliert sind, der zwischen besagten Segmenten liegt
und wobei besagte Faser weniger als 10 Gewichts-% eines Pigments oder Füllstoffs, bezogen auf das gesamte Gewicht der besagten Faser, enthält.

13. Verfahren nach Anspruch 12, wobei besagter Polyester umfasst: 50 bis 96 Mol-% eines oder mehrerer Reste von Isophthalsäure oder Terephthalsäure, bezogen auf die gesamten Säurereste; 4 bis 30 Mol-%, bezogen auf die gesamten Säurereste, eines Restes, der Natriumsulfoisophthalsäure; und 0 bis 20 Mol-%, bezogen auf die gesamten sich wiederholenden Einheiten, an Resten eines Verzweigungsmonomers mit drei oder mehr funktionellen Gruppen, wobei besagte funktionelle Gruppen Hydroxyl, Carboxyl, oder eine Kombination davon sind;
und wobei besagter geformter Querschnitt die Form einer Insel (Island-in-the-Sea-Querschnitt) oder eines Tortenstücks (Segmented-Pie-Querschnitt) hat.

14. Verfahren zum Herstellen von Microdenier-Fasern, umfassend:
(A) Spinnen der Mehrkomponentenfasern aus Anspruch 12, umfassend den wasserdispergierbaren Sulfopolyester nach Anspruch 13; und
(B) Kontaktieren der Mehrkomponentenfasern aus Schritt (A) mit Wasser, um besagten Sulfopolyester zu entfernen, wobei Microdenier-Fasern gebildet werden, welche besagte Polymere umfassen, welche in Wasser nicht dispergierbar sind.

15. Faserförmiger Gegenstand, umfassend die Microdenier-Fasern nach Anspruch 14.

16. Verfahren zum Herstellen eines Gewebes aus Microdenier-Fasern, umfassend:
(A) Spinnen der Mehrkomponentenfaser nach Anspruch 12, umfassend den wasserdispergierbaren Sulfopolyester nach Anspruch 13;
(B) Überlappen und Sammeln der Mehrkomponentenfasern aus Schritt (A), um ein Faservlies zu bilden; und
(C) Kontaktieren des besagten Faservlieses mit Wasser, um besagten Sulfopolyester zu entfernen, wodurch ein Microdenier-Faser-Gewebe gebildet wird.

## Revendications

1. Fibre multiconstituante présentant une section transversale façonnée comprenant :
A) un sulfopolyester dispersible dans l'eau ayant une température de transition vitreuse (Tg) d'au moins 57°C, ledit sulfopolyester comprenant :
(i) des résidus d'un ou plusieurs acides dicarboxyliques,
(ii) 4 à 40 % en mole, sur la base des motifs répétés totaux, de résidus d'au moins un sulfomonomère comportant 2 groupes fonctionnels et un ou plusieurs groupes sulfonate attachés sur un cycle aromatique ou cycloaliphatique dans lequel lesdits groupes fonctionnels sont hydroxyle, carboxyle ou une combinaison de ceux-ci ;
(iii) un ou plusieurs résidus de diol dans lesquels au moins 25 % en mole, sur la base des résidus de diol totaux, sont un poly(éthylène glycol) comportant une structure
H-(OCH₂-CH₂)ₙ- OH
dans laquelle n est un entier dans la plage de 2 à 500 ; et
(iv) 0 à 25 % en mole, sur la base des motifs répétés totaux, de résidus d'un monomère de ramification comportant 3 groupes fonctionnels ou plus dans lequel lesdits groupes fonctionnels sont hydroxyle, carboxyle ou une combinaison de ceux-ci ; et
B) plusieurs segments comprenant un ou plusieurs polymères non dispersibles dans l'eau, immiscibles avec ledit sulfopolyester dans lequel lesdits segments sont pratiquement isolés les uns des autres par ledit sulfopolyester intervenant entre lesdits segments ;
dans laquelle ladite fibre contient moins de 10 % en poids d'un pigment ou d'une charge, sur la base du poids total de ladite fibre.

2. Fibre selon la revendication 1, dans laquelle lesdits acides dicarboxyliques sont choisis parmi des diacides aliphatiques, des acides dicarboxyliques cycloaliphatiques, des acides dicarboxyliques aromatiques et des combinaisons de ceux-ci.

3. Fibre selon la revendication 2, dans laquelle lesdits acides dicarboxyliques sont choisis parmi succinique, glutarique, adipique, azélaïque, sébacique, fumarique, maléique, itaconique, 1,3-cyclohexanedicarboxylique, 1,4-cyclohexanedicarboxylique, diglycolique, 2,5-norbornanedicarboxylique, phtalique, téréphtalique, 1,4-naphtalènedicarboxylique, 2,5-naphtalènedicarboxylique, 2,6-naphtalènedicarboxylique, 2,7-naphtalènedicarboxylique, diphénique, 4,4'-oxydibenzoïque, 4,4'-sulfonyldibenzoïque, isophtalique et des combinaisons de ceux-ci.

4. Fibre selon l'une quelconque des revendications 1 à 3, dans laquelle ledit sulfomonomère est un sel de sulfonate métallique d'un acide sulfophtalique, d'un acide sulfotéréphtalique, d'un acide sulfoisophtalique et de combinaisons de ceux-ci.

5. Fibre selon l'une quelconque des revendications 1 à 4, dans laquelle lesdits résidus diol sont choisis parmi l'éthylène glycol, le diéthylène glycol, le triéthylène glycol, les poly(éthylène glycols), le 1,3-propanediol, le 2,4-diméthyl-2-éthylhexane-1,3-diol, le 2,2-diméthyl-1,3-propanediol, le 2-éthyl-2-butyl-1,3-propanediol, le 2-éthyl-2-isobutyl-1,3-propanediol, le 1,3-butanediol, le 1,4-butanediol, le 1,5-pentanediol, le 1,6-hexanediol, le 2,2,4-triméthyl-1,6-hexanediol, le thio-diéthanol, le 1,2-cyclohexanediméthanol, le 1,3-cyclohexanediméthanol, le 1,4-cyclohexanediméthanol, le 2,2,4,4-tétraméthyl-1,3-cyclobutanediol, le p-xylylènediol et des combinaisons de ceux-ci.

6. Fibre selon l'une quelconque des revendications 1 à 5, dans laquelle ledit monomère de ramification est le 1,1,1-triméthylolpropane, le 1,1,1-triméthyloléthane, la glycérine, le pentaérythritol, l'érythritol, le thréitol, le dipentaérythritol, le sorbitol, l'anhydride trimelllitique, le dianhydride pyromellitique, l'acide diméthylol propionique ou des combinaisons de ceux-ci.

7. Fibre selon l'une quelconque des revendications 1 à 6, dans laquelle ledit sulfopolyester comprend 50 à 96 % en mole d'un ou plusieurs résidus d'acide isophtalique ou d'acide téréphtalique, sur la base des résidus d'acide totaux ; 4 à 30 % en mole, sur la base des résidus d'acide totaux, d'un résidu d'acide sodiosulfoisophtalique ; et 0 à 20 % en mole, sur la base des motifs répétés totaux, de résidus d'un monomère de ramification comportant 3 groupes fonctionnels ou plus dans lesquels lesdits groupes fonctionnels sont hydroxyle, carboxyle ou une combinaison de ceux-ci ; et dans laquelle ladite section transversale façonnée est de la forme une ile-dans-la-mer ou une tourte segmentée.

8. Fibre selon l'une quelconque des revendications 1 à 7, dans laquelle lesdits polymères non dispersibles dans l'eau sont choisis parmi des polyoléfines, des polyesters, des polyamides, des polylactides, la polycarprolactone, le polycarbonate, le polyuréthane, le poly(chlorure de vinyle) et des combinaisons de ceux-ci.

9. Fibre selon l'une quelconque des revendications 1 à 8, dans laquelle ledit polymère non dispersible dans l'eau est biodésintégrable comme déterminé par DIN Standard 54900 ou biodégradable comme déterminé par le procédé ASTM Standard D6340-98.

10. Article fibreux comprenant la fibre selon l'une quelconque des revendications 1 à 9.

11. Article fibreux selon la revendication 10, dans lequel ledit article est choisi parmi un fil, un tissu, une bande obtenue par fusion-soufflage, une bande filée-liée, une bande thermo-liée, une bande hydro-emmêlée, un tissu non tissé et des combinaisons de ceux-ci.

12. Procédé de fabrication d'une fibre multi-constituante présentant une section transversale façonnée comprenant :
(A) le filage d'un sulfopolyester dispersible dans l'eau ayant une température de transition vitreuse (Tg) d'au moins 57°C et d'un ou plusieurs polymères non dispersibles dans l'eau, immiscibles avec ledit sulfopolyester en une fibre, ledit sulfopolyester comprenant :
(i) des résidus d'un ou plusieurs acides dicarboxyliques ;
(ii) 4 à 40 % en mole, sur la base des motifs répétés totaux, de résidus d'au moins un sulfomonomère comportant 2 groupes fonctionnels et un ou plusieurs groupes sulfonate attachés sur un cycle aromatique ou cycloaliphatique dans lequel lesdits groupes fonctionnels sont hydroxyle, carboxyle ou une combinaison de ceux-ci ;
(iii) un ou plusieurs résidus de diol dans lesquels au moins 25 % en mole, sur la base des résidus de diol totaux, sont un poly(éthylène glycol) comportant une structure
H-(OCH₂-CH₂)ₙ-OH
dans laquelle n est un entier dans la plage de 2 à 500 ; et
(iv) 0 à 25 % en mole, sur la base des motifs répétés totaux, de résidus d'un monomère de ramification comportant 3 groupes fonctionnels ou plus dans lequel lesdits groupes fonctionnels sont hydroxyle, carboxyle ou une combinaison de ceux-ci ;
dans lequel ladite fibre présente plusieurs segments comprenant lesdits polymères non dispersibles dans l'eau et lesdits segments sont essentiellement isolés les uns des autres par ledit sulfopolyester intervenant entre lesdits segments ;
et
dans lequel ladite fibre contient moins de 10 % en poids d'un pigment ou d'une charge, sur la base du poids total de ladite fibre.

13. Procédé selon la revendication 12, dans lequel ledit sulfopolyester comprend 50 à 96 % en mole d'un ou plusieurs résidus d'acide isophtalique ou d'acide téréphtalique, sur la base des résidus acide totaux ; 4 à 30 % en mole, sur la base des résidus acide totaux, d'un résidu d'acide sulfoisophtalique ; et 0 à 20 % en mole, sur la base des motifs répétés totaux, de résidus d'un monomère de ramification comportant 3 groupes fonctionnels ou plus, dans lesquels lesdits groupes fonctionnels sont hydroxyle, carboxyle ou une combinaison de ceux-ci ;
et dans lequel ladite section transversale façonnée est de la forme une ile-dans-la-mer ou une tourte segmentée.

14. Procédé de fabrication de fibres de microdenier comprenant :
(A) le filage des fibres multiconstituantes selon la revendication 12 comprenant le sulfopolyester dispersible dans l'eau selon la revendication 13 ; et
(B) la mise en contact des fibres multiconstituantes de l'étape (A) avec de l'eau pour éliminer ledit sulfopolyester formant par là des fibres de microdenier comprenant lesdits polymères non dispersibles dans l'eau.

15. Article fibreux comprenant les fibres de microdenier selon la revendication 14.

16. Procédé de fabrication d'une bande de fibres de microdenier comprenant :
(A) le filage de la fibre multiconstituante selon la revendication 12 comprenant un sulfopolyester dispersible dans l'eau selon la revendication 13 ;
(B) le chevauchement et le recueil des fibres multiconstituantes de l'étape (A) pour former une bande non tissée ; et
(C) la mise en contact de ladite bande non tissée avec de l'eau pour éliminer ledit sulfopolyester formant par là une bande de fibres de microdenier.
